# EUROPÄISCHE PATENTSCHRIFT

(11) **EP 3 889 834 B1**
(45) Veröffentlichungstag und Bekanntmachung des Hinweises auf die Patenterteilung: **01.07.2026**
(21) Anmeldenummer: 21166683.9
(22) Anmeldetag: 01.04.2021
(51) Int. Cl.: G06V 40/10, A61B 5/117, G06V 10/776, A61B 5/378, A61B 5/1171

(54) **AUTHENTIFIZIERUNGSSYSTEM AUF BASIS VON HIRNAKTIVITÄTSSIGNALEN**
AUTHENTICATION SYSTEM BASED ON BRAIN ACTIVITY SIGNALS
SYSTÈME D'AUTHENTIFICATION BASÉ SUR LES SIGNAUX D'ACTIVITÉ CÉRÉBRALE

(30) Priorität: 02.04.2020 DE 102020109284
(43) Veröffentlichungstag der Anmeldung: 06.10.2021
(73) Patentinhaber: Bundesdruckerei GmbH, 10969 Berlin (DE)
(72) Erfinder: FISCHER, Jörg, 10317 Berlin (DE); GRAUPNER, Hendrik, 14050 Berlin (DE); Dr. PAESCHKE, Manfred, 16348 Wandlitz (DE); TIETKE, Markus, 12439 Berlin (DE); TRÖLENBERG, Stefan, 15749 Mittenwalde, OT Ragow (DE)
(74) Vertreter: Richardt Patentanwälte PartG mbB

(56) Entgegenhaltungen:
- KR-B1- 101 281 852
- US-A1- 2014 020 089
- SEUL-KI YEOM ET AL: "Eeg-based person authentication using face stimuli", BRAIN-COMPUTER INTERFACE (BCI), 2013 INTERNATIONAL WINTER WORKSHOP ON, IEEE, 18 February 2013 (2013-02-18), pages 58 - 61, XP032375621, ISBN: 978-1-4673-5973-3, DOI: 10.1109/IWW-BCI.2013.6506630
- BLANCHARD NIKOLA K ET AL: "Reflexive Memory Authenticator: A Proposal for Effortless Renewable Biometrics", 25 January 2020, ADVANCES IN INTELLIGENT DATA ANALYSIS XIX; [LECTURE NOTES IN COMPUTER SCIENCE; LECT.NOTES COMPUTER], PAGE(S) 104 - 121, ISBN: 978-3-540-69901-9, ISSN: 0302-9743, XP047534545
- HAZRATI MEHRNAZ KH ET AL: "Wireless brain signal recordings based on capacitive electrodes", 2013 IEEE 8TH INTERNATIONAL SYMPOSIUM ON INTELLIGENT SIGNAL PROCESSING, IEEE, 16 September 2013 (2013-09-16), pages 8 - 13, XP032523465, DOI: 10.1109/WISP.2013.6657474

## Beschreibung

### Gebiet

Die Erfindung betrifft ein Verfahren zur Authentifizierung einer Person, insbesondere eines Authentifizierungserfahrens auf Basis biometrischer Merkmale.

### Hintergrund

Biometrische Authentifizierungsmethoden basieren auf Identifikation von Benutzern auf Basis biologischer Merkmale wie Fingerabdruck, Iris oder Stimme. Ein Scanner erfasst die biometrischen Merkmale eines Benutzers - zum Beispiel sein Irismuster oder seinen Fingerabdruck, und wandelt das erfasste biometrische Merkmal in digitale Informationen um, sodass ein Computer diese analysieren und zur Authentifizierung des Benutzers verwenden kann.

Biometrische Verfahren habe gegenüber passwortbasierten Verfahren den Vorteil, dass der Nutzer kein Passwort erinnern muss. Angesichts der Vielzahl von digitalen Identitäten, die eine Person heute im Durchschnitt verwaltet, bergen passwortbasierte Authentifizierungsverfahren die Gefahr, dass das gleiche Passwort für mehrere Accounts verwendet wird und/oder das oder die verwendeten Passwörter schriftlich oder digital festgehalten werden und bei Verlust des papierbasierten oder elektronischen Datenträgers verloren gehen oder gar in die Hände unberechtigter Dritter gelangen. Da bei biometrischen Verfahren der Nachweis der Identität durch das erbracht wird, was jemand biologisch ist, nicht dadurch, was jemand weiß, muss auch kein Passwort erinnert oder sicher verwahrt werden.

Allerdings haben biometrische Verfahren den Nachteil, dass das Authentifizierungsverfahren nicht einfach zurückgesetzt werden kann (analog zu dem Ändern eines Passworts). Biometrische Daten können gestohlen werden. Auf der Basis von Fingerabdrücken, die auf Gegenständen wie Türklinken oder Trinkgläsern vielfach hinterlassen werden, können Folien bzw. Kunststoffreplika erzeugt werden, die den auf Gegenständen hinterlassenen Fingerabdruck akkurat widergeben und von unberechtigten Dritten dazu verwendet werden können, sich erfolgreich zu authentifizieren. Mittlerweile ist bekannt, dass noch nicht einmal ein physischer Fingerabdruck verfügbar sein muss, um diesen ein gefälschtes Fingerabdruckreplika zu erstellen. Ein hochauflösendes Bild des Fingerabdrucks, das irgendwo online gespeichert ist, reicht für die Erstellung dieses Replikas aus.

Wenn biometrische Daten kompromittiert werden, können die Folgen weitreichend sein, da man sie nicht zurücksetzen kann. Ein Fingerabdruck oder Blutgefäßmuster lässt sich nur bedingt oder gar nicht ändern. Wenn ein Kennwort kompromittiert wird, kann es dagegen problemlos zurückgesetzt werden.Yeom et al. (2013) [Yeom, S., Suk, H., & Lee, S. (2013). Person authentication from neural activity of facespecific visual self-representation., 1159-1169.] beschreiben ein neues biometrisches System, das auf den neurophysiologischen Merkmalen der gesichtsspezifischen visuellen Selbstrepräsentation im menschlichen Gehirn basiert, die mittels Elektroenzephalografie (EEG) gemessen werden können. Das Verfahren umfasst ein Stimulus-Präsentationsparadigma, das Selbst- und Nicht-Selbst-Gesichts-Bilder als Stimuli für ein Personen-Authentifizierungssystem verwendet, das die Identität einer Person durch den Vergleich eines beobachteten Merkmals mit den in der Datenbank gespeicherten Merkmalen validieren kann (Eins-zu-Eins-Abgleich). Das beschriebene Paradigma erzeugt einzigartige subjektspezifische Hirnwellenmuster als Reaktion auf Selbst- und Nicht-Selbst-Gesichtsbilder.

Die Druckschrift KR 101 281 852 B1 beschreibt ein Verfahren zur Authentifizierung unter Verwendung von Hirnaktivitätssignalen. Erste und zweite Hirnaktivitätssignale werden gemessen, während Selbst- und Nicht-Selbst-Gesichtsbilder angezeigt werden. Beim Auftreten von Maxima zwischen den ersten und zweiten Hirnaktivitätssignalen werden die ersten und zweiten Hirnaktivitätssignale miteinander verglichen. Sich dabei ergebende Differenzwerte werden mit Differenzwerten aus einer Datenbank abgeglichen. Die wissenschaftliche Veröffentlichung SEUL-KI YEOM ET AL: "Eeg-based person authentication using face stimuli", BRAIN-COMPUTER INTERFACE (BCI), 2013 INTERNATIONAL WINTER WORKSHOP ON, IEEE, 18. Februar 2013, Seiten 58-61, DOI: 10.1109/IWW-BCI.2013.6506630, ISBN 978-1-4673-5973-3 beschreibt ein Verfahren, welches ebenfalls erste und zweite Hirnaktivitätssignale bei der Betrachtung von Selbst- und Nicht-Selbst-Gesichtsbildern vergleicht.

### Zusammenfassung

Der Erfindung liegt die Aufgabe zugrunde, ein verbessertes Authentifizierungsverfahren vorzuschlagen, sowie ein entsprechendes Authentifizierungssystem.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen sind frei miteinander kombinierbar, sofern sie sich nicht gegenseitig ausschließen.

In einem Aspekt betrifft die Erfindung ein Verfahren zur Authentifizierung einer Person. Das Verfahren umfasst:
- Bereitstellung einer Anzeigevorrichtung;
- Bereitstellung eines kontaktlosen Sensors zur Sensierung von Hirnaktivitäten - im Folgenden als KLHA-Sensor bezeichnet;
- Anzeige mehrerer Gesichtsbilder, die Gesichter mehrerer unterschiedlicher Personen abbilden, auf der Anzeige für die eine Person; die mehreren Gesichtsbilder umfassen ein oder mehrere Selbstbilder und ein oder mehrere Fremdbilder; ein Selbstbild ist ein Gesichtsbild dieser einen Person; ein Fremdbild ein Gesichtsbilder einer anderen der mehreren unterschiedlichen Personen;
- während die mehreren Gesichtsbilder der einen Person angezeigt werden, Erfassung von ersten und zweiten Hirnaktivitätssignalen, die in der einen Person durch die Betrachtung der Gesichtsbilder jeweils ausgelöst werden, durch den KLHA-Sensor, wobei die ersten Hirnaktivitätssignale erfasst werden, während dieser einen Person die ein oder mehreren Selbstbilder angezeigt werden, und wobei die zweiten Hirnaktivitätssignale erfasst werden, während dieser einen Person die ein oder mehreren Fremdbilder angezeigt werden;
- Analyse der erfassten ersten und zweiten Hirnaktivitätssignale durch eine Authentifizierungssoftware, um die eine Person gegenüber der Authentifizierungssoftware zu authentifizieren, wobei die Authentifizierungssoftware die eine Person nur dann als erfolgreich authentifiziert behandelt, wenn die ersten Hirnaktivitätssignale sich signifikant unterscheiden von den zweiten Hirnaktivitätssignalen, und wenn neben der signifikanten Unterschiedlichkeit der ersten und zweiten Hirnaktivitätssignale folgende weitere Kriterien zutreffen:
- alle ersten Hirnaktivitätssignale, die je während des Anzeigens eines der Selbstbilder erfasst werden, sind identisch oder hinreichend ähnlich zueinander;
- alle zweiten Hirnaktivitätssignale, die je während des Anzeigens eines der Fremdbilder erfasst werden, sind identisch oder hinreichend ähnlich zueinander.

Dieses Verfahren kann aus mehreren Gründen vorteilhaft sein: Da die eine Person nur dann als authentifiziert behandelt wird, wenn die ersten Hirnaktivitätssignale untereinander identisch oder hinreichend ähnlich sind und wenn zudem die zweiten Hirnaktivitätssignale untereinander identisch oder hinreichend ähnlich sind, ist die Authentifizierung besonders sicher. Der Erfindung liegt somit insbesondere die Aufgabe zugrunde, ein Verfahren anzugeben, das ein höheres Sicherheitsniveau bei der Authentifizierung bereitstellt. Außerdem wird das Problem überwunden, dass manche biometrischen Daten gestohlen werden können und die gestohlenen Daten es Unberechtigten ermöglichen, sich mit diesen erfolgreich zu authentifizieren. Die biometrischen Daten, mit welchen sich eine Person authentifiziert, hängen nämlich nicht nur von Merkmalen ab, die intrinsisch an den Körper der Person gekoppelt sind, sondern zudem auch davon, welche Art von Bildern der zu authentifizierenden Person zu einem bestimmten Zeitpunkt gezeigt werden. Personen hinterlassen bei fast jeder alltäglichen Aktivität Fingerabdrücke und es ist für Angreifer entsprechend einfach, den Fingerabdruck einer Person von Gegenständen, Türgriffen oder ähnlichen zu erfassen und zur Herstellung gefälschter Fingerabdruck-Replika nutzen. Die ersten und zweiten Hirnaktivitätssignale, die gemäß Ausführungsformen der Erfindung zur Authentifizierung einer Person dienen, entstehen jedoch in Antwort auf das Anzeigen bzw. das betrachten einer bestimmten Sequenz von Selbstbildern und Fremdbildern. Das Erfassen von Hirnaktivitätssignalen im Alltag der Person wird einen Angreifer nicht in die Lage versetzen, sich durch das Ausstrahlen von heimlich im Alltagsleben einer Person erfassten Hirnaktivitätssignalen dieser einen Person zu authentifizieren, denn die Hirnaktivitätssignale, die entstehen, wenn eine Person fernsieht, sich im Raum bewegt, mit anderen Leuten spricht oder isst unterscheiden sich von den ersten und zweiten Hirnaktivitätssignale, die entstehen, wenn dieser einen Person Selbstbilder und Fremdbilder gezeigt werden.

Sogar wenn es einem Angreifer gelänge, die ersten und zweiten Hirnaktivitätssignale der Person zu messen, auf deren Basis sich die eine Person erfolgreich gegenüber der Authentifizierungssoftware authentifiziert hat, besteht eine erhebliche technische Schwierigkeit darin, diese Signale über eine technische Sendeeinheit so auszustrahlen, dass sie auf den Sensor wirken wie die echten Hirnaktivitätssignale einer Person.

Und sogar falls der Angreifer eine solche Sendeeinheit erfolgreich hergestellt und in die Räumlichkeiten mit der Anzeige eingeschleust hat, würde dies allein dem Angreifer noch nicht ermöglichen, sich gegenüber der Authentifizierungssoftware zu authentifizieren, denn die Art der gefälschten Hirnaktivitätssignale, die von der Sendeeinheit des Angreifers ausgesendet wird, müsste mit der Art der Gesichtsbilder, die dem Angreifer gezeigt wird, synchronisiert werden. Falls die Reihenfolge der angezeigten Gesichtsbilder abweicht von der Reihenfolge der Anzeige beim unberechtigten Aufzeichnen der Hirnsignale durch den Angreifer wird der Authentifizierungsversuch des Angreifers fehlschlagen. Gleiches gilt, falls der Zeitpunkt des Beginns des Sendens der Hirnsignale nicht zeitgleich ist zu dem Beginn des Anzeigens der Gesichtsbilder. Außerdem würde auch das Gehirn des "Angreifers" beim Anblick der Gesichtsbilder Hirnaktivitätssignale aussenden, die sich mit den Signalen der technischen Sendeeinheit des Angreifers überlagern würden, sodass allein schon deshalb sichergestellt ist, dass das bei dem Sensor ankommende und erfasste Signal nicht identisch ist zu den ersten und zweiten Hirnaktivitätssignalen, die zu einer erfolgreichen Authentifizierung führen würden.

Ein weiterer Vorteil kann die erhöhte Nutzerfreundlichkeit des Authentifizierungsverfahrens sein. Man muss sich beispielsweise kein Passwort merken.

Ein weiterer Vorteil besteht darin, dass das Verfahren sicher ist bezüglich eines Authentifizierungsversuchs eines unberechtigten Dritten, der eine Gesichtsmaske trägt die aussieht wie das Gesicht einer berechtigten Person, oder der Kontaktlinsen trägt, deren Irismuster aussieht wie das Irismuster einer berechtigten Person, oder die ein sonstiges Replika eines biometrischen Merkmals einer berechtigten Person mit sich führt. Beispielsweise ist es denkbar, dass eine unberechtigte Person eine Gesichtsmaske trägt, sodass die Maske der unberechtigten Person dem Gesicht nach ein Aussehen verleiht, das so ähnlich zu dem Gesicht einer berechtigten Person ist, dass weder ein menschlicher Kontrolleur noch eine Gesichtserkennungssoftware die Maske als solche erkennt. Wird dieser eigentlich unberechtigten Person mit der Maske allerdings im Zuge der Authentifizierung das Bild der eigentlich berechtigten Person angezeigt (das zum Beispiel in einer Mitarbeiter Datenbank hinterlegt ist und/oder das auf einem gefälschten Ausweis dieser Person abgedruckt ist), dann wird der Sensor anstatt der zu erwartenden ersten Hirnaktivitätssignale, die bei Anblick eines Selbstbilds entstehen, zweite Hirnaktivitätssignale, die beim Anblick eines Fremdbilds entstehen, erfassen. Ein Betrüger kann zwar mithilfe der Maske und eines gefälschten Personalausweises vorübergehend das Aussehen einer eigentlich berechtigten Person annehmen, er kann aber nicht verhindern, dass seine eigenen Hirnsignale beim Betrachten eines Gesichtsbildes dieser berechtigten Person zu erkennen geben, dass die in diesem abgebildete Person eine andere ist als die, die sich eben versucht zu authentifizieren.

Ein weiterer Vorteil besteht in dem Schutz vor einer Authentifizierung einer unberechtigten Person auf Basis eines gemorphten Bildes auf einem Ausweis, z.B. einem Reisepass oder Personalausweises. Mittlerweile gibt es Softwareprogramme, die biometrische Fotos von zwei Personen "morphen", also miteinander auf elektronischem Wege zu einem einzigen "gemorphten" Bild kombinieren können. Im Ergebnis zeigt das gemorphte Bild biometrische Merkmale zweier Personen, sodass die Gefahr besteht, dass eine berechtigte Person im Zuge der Ausweiserstellung ein gemorphtes Bild abgibt, sodass sich auf Basis des neuen Ausweises mit dem gemorphten Bild neben der eigentlich berechtigten Person noch eine weitere Person mit diesem Ausweis authentifizieren kann. Die Anmelderin hat jedoch beobachtet, dass eine Person, deren Gesichtsbild für die Erstellung eines gemorphten Bildes verwendet wurde, aber sehr wohl erkennt, dass dieses Bild erheblich von einem echten eigenen Bild (Selbstbild) abweicht. Die Hirnaktivitätssignale einer Person, die ein gemorphtes Bild ihres eigenen Gesichtsbilds betrachtet, sind also den Hirnaktivitätssignalen, die beim Betrachten eines Fremdbildes entstehen, recht ähnlich, sodass die Authentifizierungssoftware gemäß Ausführungsformen der Erfindung einen Authentifizierungsversuch auf Basis eines Ausweises mit gemorphtem Gesichtsbild erkennen und eine Authentifizierung verweigern wird.

Ein weiterer Vorteil besteht darin, dass die Erfassung der Hirnaktivitätssignale kontaktlos erfolgt. Das Authentifizierungsverfahren gemäß Ausführungsformen der Erfindung ist somit besonders geeignet zur schnellen Authentifizierung einer großen Anzahl von Personen, zum Beispiel im Kontext von Passagierkontrollen an Flughäfen, Personenkontrollen an Grenzübergängen oder Personenkontrollen im Eingangsbereich von Firmengeländen. Viele biometrische Authentifizierungsverfahren erfordern einen physischen Kontakt des sich authentifizierenden Person mit dem Sensor, zum Beispiel zur Erfassung von Fingerabdrücken. Dies erfordert Zeit und reduziert die Nutzbarkeit mancher biometrische Authentifizierungsverfahren in Anwendungsszenarien in welchen mit hohem Personenaufkommen zu rechnen ist. Demgegenüber ermöglicht eine kontaktlose Erfassung der Hirnaktivitätssignale eine sehr schnelle Erfassung der biometrischen Merkmale einer Person.

Gegenüber dem von Yeom et al. (2013) beschriebenen Verfahren und anderen aus dem Stand der Technik bekannten Verfahren können Ausführungsformen der Erfindung mehrere Vorteile aufweisen: Das Verfahren ist noch sicher gegenüber einem Angreifer, der sich fälschlicherweise als eine andere Person zu authentifizieren versucht, denn das Verfahren beruht nicht notwendigerweise auf dem Vergleich eines beobachteten Merkmals mit dem in einer Datenbank gespeicherten Merkmal, sondern auf dem direkten Vergleich von Hirnaktivitätssignalen, die in Reaktion auf Selbst- bzw. Fremdbilder erzeugt wurden. Das Verfahren benötigt außerdem nicht die Speicherung von Merkmalen einzelner Personen in einer Datenbank, wodurch die Privatsphäre der zu authentifizierenden Person geschützt wird. Hierdurch kann das Verfahren auch in Situationen eingesetzt werden bei denen eine Speicherung personenbezogener, biometrischer Daten unerwünscht ist. Durch die Löschung der Daten kann außerdem die Privatsphäre der sich authentifizierenden Person verbessert werden ("improved privacy").

Nach Ausführungsformen umfasst das Anzeigen der mehreren Gesichtsbilder für jeden Authentifizierungsvorgang eine Erzeugung einer zufälligen Reihung der mehreren Gesichtsbilder durch die Authentifizierungssoftware und Durchführen des Anzeigens der mehreren Gesichtsbilder in einer chronologischen Sequenz entsprechend der zufälligen Reihung.

Beispielsweise kann die Authentifizierung Software dazu konfiguriert sein, jeder Person, die sich authentifizieren möchte, eine Sequenz aus sechs Gesichtsbildern anzuzeigen. Die sechs Gesichtsbilder können zum Beispiel immer eine festgelegte Zahl an Selbstbildern und Fremdbildern umfassen, zum Beispiel immer zwei Selbstbilder und 4 Fremdbilder. Es ist aber auch möglich, dass der relative Anteil von Fremdbildern und Selbstbildern bei jedem Authentifizierungsverfahren ebenfalls zufällig neu gewählt wird, solange nur sichergestellt ist, dass die mehreren Gesichtsbilder mindestens ein Selbstbild und mindestens ein Fremdbild beinhalten.

Nach Ausführungsformen der Erfindung dient die für jeden Authentifizierungsprozess erzeugte zufälligen Reihung als Challenge. Die Analyse der erfassten ersten und zweiten Hirnaktivitätssignale umfasst eine Prüfung, ob die zeitliche Reihenfolge der ersten und zweiten Hirnaktivitätssignale die in der Challenge codierte Reihenfolge von Selbstbildern und Fremdbildern widergibt. Nur in diesem Fall behandelt die Authentifizierungssoftware den einen Nutzer als erfolgreich authentifiziert.

Dies kann vorteilhaft sein, da hierdurch ein besonders wirksamer Schutz gegen Replay-Angriffen erreicht werden kann.

Nach Ausführungsformen behandelt die Authentifizierungssoftware die eine Person nur dann als erfolgreich authentifiziert, wenn neben der signifikanten Unterschiedlichkeit der ersten und zweiten Hirnaktivitätssignale folgende weitere Kriterien zutreffen: alle ersten Hirnaktivitätssignale, die je während des Anzeigens eines der Selbstbilder erfasst werden, sind identisch oder hinreichend ähnlich zueinander; und alle zweiten Hirnaktivitätssignale, die je während des Anzeigens eines der Fremdbilder erfasst werden, sind identisch oder hinreichend ähnlich zueinander.

Der Ausdruck "identisch oder ähnlich" kann insbesondere bedeuten, "identisch im Rahmen der jeweiligen Messungenauigkeit des Sensors oder so ähnlich, dass ein vordefiniertes Mindestähnlichkeitskriterium erfüllt ist". Bei dem vordefinierten Mindestähnlichkeitskriterium kann es sich z.B. einen vordefinierten Mindestwert für ein Ähnlichkeitsmaß (Kurvenabstand, Integral der Differenzfläche der Hirnaktivitätssignalprofile über einen Zeitraum, etc.) handeln.

Zusätzlich oder alternativ dazu umfasst das Anzeigen der mehreren Gesichtsbilder für jeden Authentifizierungsvorgang eine zufällige Auswahl zumindest der ein oder mehreren Fremdbilder und zusätzlich optional auch der ein oder mehreren Selbstbilder aus einer Gesichtsbilddatenbank durch die Authentifizierungssoftware und ein Durchführen des Anzeigens der mehreren ausgewählten Gesichtsbilder einschließlich des Selbstbilds.

Beispielsweise kann es sich bei der Bilddatenbank um eine Datenbank mit Gesichtsbildern von Mitarbeitern einer Firma oder sonstigen Organisation handeln. Beispielsweise kann die Person, die sich authentifizieren möchte, ihren Namen eingeben oder die Authentifizierung Software erkennt auf andere Weise, zum Beispiel durch automatische Gesichtserkennung, welche aus einer Vielzahl von registrierten Personen (zum Beispiel Mitarbeitern) sich eben authentifizieren möchte. Auf Basis dieser Information durchsucht die Authentifizierungssoftware die Bilddatenbank und ermittelt ein oder mehrere Selbstbilder, die in der Datenbank verknüpft mit dem eingegebenen Namen bzw. automatisch erkannten Identifikator dieser Person gespeichert sind. Außerdem ermittelt die Authentifizierungssoftware ein oder mehrere Fremdbilder, die Gesichter von anderen Personen abbilden, die nicht die Person darstellen, die sich eben authentifizieren möchte. Bei den anderen Personen kann es sich um andere Mitarbeiter bzw. Mitglieder der Organisation handeln, die sich im Prinzip mittels dieser Bilder authentifizieren könnten. In diesem Fall kann ein bestimmtes Gesichtsbild sowohl als Selbstbild als auch als Fremdbild von der Authentifizierungssoftware verwendet werden je nachdem, welche Person sich gerade authentifizieren möchte. In anderen Ausführungsformen zeigen die in der Datenbank gespeicherten Fremdbilder Personen, die nicht Mitglied der besagten Firma bzw. Organisation sind, die also niemals bei einem Authentifizierungsverfahren für diese Organisation als Selbstbild dienen können, da sie nicht das Gesicht einer registrierten Person bzw. eines registrierten Organisationsmitglieds abbilden. Die Verwendung von Gesichtsbildern von Nicht-registrierten Personen als Fremdbilder hat den Vorteil, dass sichergestellt ist, dass das in einem Fremdbild dargestellte Gesicht der sich authentifizieren Person sicherlich nicht bekannt ist, wohingegen im Hinblick auf Gesichtsbilder von Mitarbeitern im Einzelfall ein Wiedererkennungseffekt in den Hirnaktivitätssignalen erkennbar sein kann, wenn es sich zum Beispiel um einen Kollegen handelt, dem der Mitarbeiter täglich begegnet. Die Verwendung von Gesichtsbildern anderer bei der Organisation registrierter Personen als Fremdbilder kann den Vorteil haben, dass automatisch eine recht große Menge unterschiedlicher Gesichtsbilder verfügbar ist.

Die Authentifizierungssoftware ist dazu ausgebildet, die eine Person nur dann als authentifiziert zu behandeln, wenn die ersten Hirnaktivitätssignale sich signifikant unterscheiden von den zweiten Hirnaktivitätssignalen, und wenn zugleich die ersten Hirnaktivitätssignale untereinander identisch oder hinreichend ähnlich sind und wenn zudem die zweiten Hirnaktivitätssignale untereinander identisch oder hinreichend ähnlich sind. Hinreichend ähnlich kann z.B. bedeuten, dass ein Ähnlichkeitswert berechnet wird und die verglichenen Signale nur dann als hinreichend ähnlich angesehen werden, wenn dieser Ähnlichkeitswert über einem vordefinierten Mindestwert liegen. Der Mindestwert kann vom Typ des jeweiligen Signals abhängen und z.B. empirisch im Vorfeld mit mehreren Testpersonen ermittelt werden.

Nach Ausführungsformen besteht die Anzeige aus einer Vorrichtung mit einem oder mehreren Spiegeln oder die Anzeige umfasst diese Vorrichtung. Die Vorrichtung ist dazu ausgebildet, ein Spiegelbild des Gesichts der einen Person oder ein Spiegelbild eines auf einem ID-Dokument der einen Person abgebildeten Gesichtsbildes optisch so zu erfassen und abzulenken, dass dieses Spiegelbild dieser Person als eines der ein oder mehreren Selbstbilder angezeigt wird. Unter einem Spiegel wird hierbei ein breites Spektrum von Vorrichtungen zum Spiegeln verstanden, wobei Kameras, digitale Vorrichtungen zum Spiegeln, Displays, Web-Cams und Spiegelfolie ebenfalls zu den Spiegeln gezählt werden.Dies kann vorteilhaft sein, da diese Ausführungsform auf besonders sichere Weise davor schützt, dass sich ein unberechtigter mit einer Maske und/oder einen gefälschten Personalausweis authentifiziert: sogar wenn die Maske das Gesicht eines berechtigten Mitarbeiters täuschend echt nachbildet und/oder wenn das Gesichtsbild auf dem gefälschten Ausweis dem Gesicht des berechtigten Mitarbeiters täuschend ähnlich ist, wird der Anblick der Maske bzw. des Ausweis-Bildes im Spiegelbild in dem Betrachter nicht die für die Betrachtung von Selbstbildern typischen ersten Hirnaktivitätssignale hervorrufen. Außerdem ist in den Ausführungsformen, in welchen die Spiegel so angebracht sind, dass der Person das eigene Spiegelbild als Selbstbild angezeigt wird, automatisch sichergestellt, dass die als Selbstbild dienenden Gesichtsbilder stets aktuell sind, denn die Person sie dir das eigene Spiegelbild und nicht ein vor gegebenenfalls langer Zeit aufgenommenes und in einer Datenbank gespeichertes Foto. Um in dieser Ausführungsform eine zufällige Sequenz mehrerer Selbstbilder und Fremdbilder anzuzeigen können beispielsweise der Ausweis (oder ein Foto) der zu authentifizierenden Person und Ausweise (oder Fotos) von anderen Personen gemäß dieser zufälligen Sequenz in die Vorrichtung an einer bestimmten Position eingebracht werden, sodass die Spiegelvorrichtung das Spiegelbild des Ausweises bzw. des Fotos der zu authentifizierenden Person angezeigt wird. Es ist auch möglich, dass die Spiegelvorrichtung neben den ein oder mehreren Spiegeln auch eine Digitalanzeige beinhaltet und/oder dass es sich bei einem der Spiegel um einen semi-transparenten Spiegel handelt. Die Vorrichtung und die darin enthaltenen Spiegel sind so dimensioniert, dass eine Person beim Betreten der Vorrichtung zunächst das eigene Spiegelbild sieht, dieses Spiegelbild jedoch gemäß der zufälligen Sequenz ersetzt wird durch ein Fremdbild, das über die Digitalanzeige angezeigt wird. Beispielsweise kann der Spiegel ein semitransparenter Spiegel sein, der eine Digitalanzeige überlagert. Wenn die Person eine Selbstbild sehen soll, wird innerhalb der Vorrichtung eine Beleuchtung eingeschaltet und optional darüber hinaus die Digitalanzeige ausgeschaltet bzw. dazu veranlasst, nur ein schwarzes Bild anzuzeigen. Das Spiegelbild, dass die Person dann von sich selbst im semi-transparenten Spiegel sieht, entspricht also dem eigenen Spiegelbild. Wenn die Person ein Fremdbild sehen soll, wird die Beleuchtung ausgeschaltet und ein digitales Bild einer anderen Person auf der digitalen Anzeige angezeigt. Da die digitale Anzeige nun die einzige Lichtquelle ist, ist ihr Licht ausreichend, sodass die sich authentifizierende Person durch den semi-transparenten Spiegel nur das Fremdbild innerhalb der Digitalanzeige sieht, nicht das eigene Spiegelbild.

Die Verwendung einer Vorrichtung mit einer oder mehreren Spiegeln kann den Vorteil haben, dass sie vor einer Manipulation der Bilddatenbank schützt. Wenn nämlich die Bilddatenbank gehackt wird und die Gesichtsbilder, die einer bestimmten, berechtigten Person in der Datenbank zugewiesen sind, durch die Gesichtsbilder einer unberechtigten Person ersetzt werden, kann es dieser unberechtigten Person gelingen, sich zu authentifizieren, denn die Anzeige dieser manipulierten Bilder wird bei der nicht berechtigten Person die für Selbstbilder typischen ersten Hirnaktivitätssignale erzeugen. Wird das Selbstbild jedoch als Spiegelbild innerhalb einer Vorrichtung mit einem oder mehreren Spiegeln erzeugt, wird der Person zwingend ihr eigenes Spiegelbild als Selbstbild gezeigt. Wenn die Person eine Maske trägt, wird sie nicht die für Selbstbilder typischen ersten Hirnaktivitätssignale emittieren. Wenn die Person keine Maske trägt, wird sie in jedem Fall die ersten Hirnaktivitätssignale emittieren, die sich dann in signifikanter Weise von den Hirnaktivitätssignalen unterscheiden, die beim Betrachten von Fremdbildern erfasst werden.

Gemäß Ausführungsformen wird das hier beschriebene Authentifizierungsverfahren kombiniert mit weiteren, für sich alleine genommen möglicherweise nicht sicheren Verfahren, eine Person sicher zu authentifizieren. Beispielsweise umfasst das Authentifizierungsverfahren gemäß einer Ausführungsform zusätzlich eine automatische Authentifizierung der einen Person durch eine Gesichtserkennungssoftware. Authentifizierungsverfahren rein auf Basis von Gesichtserkennungssoftware sind unsicher, da bereits eine Maske ausreicht, dass Unberechtigte sich erfolgreich authentifizieren können. In Kombination jedoch mit dem beschriebenen Authentifizierungsverfahren unter Verwendung der Vorrichtung mit einem oder mehreren Spiegeln ist es ausgeschlossen, dass sich ein Unberechtigter mit einer Maske erfolgreich authentifizieren kann, denn auch bei einer täuschend echten Maske würde das beim Betrachten des eigenen Spiegelbilds erzeugte Hirnaktivitätssignal den Hirnaktivitätssignalen für Fremdbilder gleichen.

Nach Ausführungsformen besteht die Anzeige aus einer digitalen Anzeige oder umfasst eine digitale Anzeige. Die digitale Anzeige ist dazu ausgebildet, die mehreren Gesichtsbilder als digitale Bilder anzuzeigen. Beispielsweise können sowohl die Fremdbilder als auch die Selbstbilder als Gesichtsbilder von Personen ausgebildet sein, die als digitale Bilder in einer Datenbank gespeichert sind.

Die Verwendung einer digitalen Anzeige hat den Vorteil der größeren apparativen Einfachheit. Digitale Anzeigen in Form von z.B. LCD-, LED- oder OLED-Bildschirmen gibt es in allen möglichen Größen und Formen und zu günstigen Preisen. Oftmals sind bestehende, aktuell schon für Authentifizierungszwecke verwendete Terminals ohnehin schon mit einer digitalen Anzeige ausgestattet.

Nach Ausführungsformen umfasst das Verfahren eine Bereitstellung des Selbstbildes für die Anzeige des Selbstbildes als eines der mehreren Gesichtsbilder. Die Bereitstellung umfasst:
- Erfassung und Ablenkung eines Spiegelbilds des Gesichts der einen Person durch eine Vorrichtung mit einem oder mehreren Spiegeln, sodass dieses Spiegelbild dieser Person als eines der ein oder mehreren Selbstbilder angezeigt wird; oder
- Erfassung und Ablenkung eines Spiegelbilds eines auf einem ID-Dokument der einen Person abgebildeten Gesichtsbildes durch eine Vorrichtung mit einem oder mehreren Spiegeln, sodass dieses Spiegelbild dieser Person als eines der ein oder mehreren Selbstbilder angezeigt wird; oder
- Erfassung des Gesichts der einen Person oder eines auf einem ID-Dokument der einen Person abgebildeten Gesichtsbildes durch eine Kamera in Form eines digitalen Gesichtsbildes der einen Person.

Die vor und Nachteile der unterschiedlichen Arten, die das Selbstbild einer Person erfasst und angezeigt werden kann, wurden oben bereits erwähnt. Die Verwendung von Spiegeln ist im allgemeinen apparativ aufwendiger, schützt aber wirksam vor einer unberechtigten Authentifizierung Dritter, die eine Maske tragen und zuvor möglicherweise die Bilddatenbank manipuliert haben. Die Verwendung von Digitalanzeigen ist günstig und kann oftmals auf Basis bereits bestehender Hardware bzw. Terminalinfrastruktur durchgeführt werden. Die Erfassung von Gesichtsbildern auf Ausweisen wie zum Beispiel Reisepässen, Personalausweisen oder Mitarbeiterausweisen kann vorteilhaft sein, da diese Dokumente oftmals weitere Sicherheitsmerkmale und/oder personenbezogene Daten beinhalten, die im Zuge des Authentifizierungsverfahrens ohnehin analysiert und ausgewertet bzw. ausgelesen werden müssen. In diesem Fall kann die Überprüfung der unterschiedlichen Hirnaktivitätssignale bei der Betrachtung von Selbstbildern und Fremdbildern als zusätzliche Sicherheitskomponente im Kontext eines noch komplexeren Authentifizierungsverfahrens verwendet werden , innerhalb welchen ein oder mehrere weitere Sicherheitsmerkmale des Dokuments wie zum Beispiel Hologramme, Prüfzahlen, kryptographische Schlüssel oder dergleichen ausgewertet werden.

Nach Ausführungsformen umfassen die mehreren Gesichtsbilder mindestens zwei Selbstbilder. Die mindestens zwei Selbstbilde werden auf unterschiedliche Weise erfasst, wobei die Erfassungsweisen insbesondere ausgewählt sind aus den oben genannten verschiedenen Erfassungsweisen. Die eine Person wird nur dann als erfolgreich authentifiziert angesehen, wenn die ersten Hirnaktivitätssignale, die erfasst wurden, während dieser einen Person die mindestens zwei Selbstbilder angezeigt wurden, sich auf signifikante Weise unterscheiden von den zweiten Hirnaktivitätssignalen.

Ein Vorteil des Authentifizierungsverfahrens gemäß Ausführungsformen der Erfindung besteht auch darin, dass es nicht notwendig ist, dass die ersten Hirnaktivitätssignale bei verschiedenen Personen identische oder nahezu identische Eigenschaften aufweisen. Die Anmelderin hat beobachtet, dass auch dann, wenn die durch Betrachtung von Selbstbildern ausgelösten ersten Hirnaktivitätssignale von Person zu Person deutlich unterschiedlich sind, sie dann doch zumindest sehr deutlich unterschiedlich sind von den zweiten Hirnaktivitätssignalen, die beim Betrachten von Fremdbildern erzeugt werden. Unter der Voraussetzung dass der zu authentifizieren Person eine hinreichend große Anzahl von Fremdbildern und Selbstbildern gezeigt wird, ist eine Analyse der zeitlichen Abfolge der Eigenschaften der Hirnaktivitätssignale während der Anzeigezeit ausreichend. Beispielsweise können der zu authentifizierenden Person mindestens zwei, vorzugsweise mindestens 3 Selbstbilder und mindestens zwei, vorzugsweise mindestens 5 Fremdbilder angezeigt werden. Die Hirnaktivitätssignale der Person werden während der Anzeige der verschiedenen Selbstbilder und Fremdbilder kontinuierlich erfasst und mit der bekannten zeitlichen Sequenz der Anzeige der Fremdbilder und Selbstbilder verglichen. Stellt sich heraus, dass die ersten Hirnaktivitätssignale, die erfasst wurden, während die eine Person die Selbstbilder betrachtete, alle identische oder sehr ähnliche Eigenschaften haben, und dass die zweiten Hirnaktivitätssignale, die erfasst wurden, während die eine Person die Fremdbilder betrachtete, alle identische oder sehr ähnliche Eigenschaften haben, wobei die Eigenschaften der ersten und zweiten Hirnaktivitätssignale signifikant unterschiedlich sind, gilt die eine Person gemäß Ausführungsformen der Erfindung als erfolgreich authentifiziert.

Die von dem KLHA-Sensor erfassten Hirnaktivitätssignale können zum Beispiel drahtgebunden (zum Beispiel per Ethernet-Kabel) oder über eine drahtlose Schnittstelle (zum Beispiel Bluetooth) an die Authentifizierung Software übertragen werden.

Die Authentifizierungssoftware kann dazu ausgebildet sein, die Hirnaktivitätssignale, die von dem KLHA-Sensor empfangen werden, zu korrelieren mit weiteren Messdaten. Beispielsweise kann eine Kamera die Position und/oder Orientierung des Kopfes der einen Person im Raum erkennen und an die Authentifizierung Software weiterleiten. Änderungen der Position und/Orientierung des Kopfes können also mit Signaländerungen korreliert werden. Dies kann hilfreich sein, denn nach Ausführungsformen der Erfindung verwendet die Authentifizierungssoftware diese weiteren Messdaten, um Störsignale, die durch Bewegung des Kopfes im Raum während der Authentifizierung erzeugt werden, herauszufiltern, sodass nur oder vorwiegend diejenigen Messsignale übrig bleiben, die ursächlich auf die Betrachtung von Fremdbildern und Selbstbildern bzw. auf die Beobachtung eines Bildwechsels zurückzuführen sind. Auch Vibrationen des Sensors, die darauf beruhen, dass Vibrationen, die z.B. durch Geh- und Dreh-Bewegungen der einen Person im Raum während des Authentifizierungsprozesses erzeugt werden und durch mechanische Kopplung an den Sensor übertragen werden, können bei manchen Sensortypen automatisch erfasst werden, z.B. wenn der Sensor als elektrischer Sensor ausgebildet ist. Zusätzlich oder alternativ dazu kann die Kamera eine Serie von Gesichtsbildern der einen Person über einen Zeitraum erfassen und diese Gesichtsbilder an die Authentifizierungssoftware weiterleiten. Durch Analyse der zeitlichen Serie der Gesichtsbilder kann die Authentifizierungssoftware beispielsweise erkennen, ob und wann die eine Person mit den Augen blinzelt und/oder ob und wann die Person eine deutliche Emotion (Freude, Angst, Schreck, Unsicherheit) erlebt, die zum Beispiel anhand des Gesichtsausdrucks erkennbar ist. Hirnaktivitätssignale, die mit solchen Emotionen einhergehen, können die durch die Betrachtung der Selbstbilder und Fremdbilder erzeugten Hirnaktivitätssignale überlagern und zu Fehlern führen. Auch die mit starken Emotionen einhergehenden Hirnaktivitätssignale werden gemäß Ausführungsformen der Erfindung von der Authentifizierungssoftware automatisch als Störsignale erkannt und herausgefiltert.

Nach Ausführungsformen der Erfindung wird die Analyse ausschließlich auf Basis der ersten und zweiten Hirnaktivitätssignale und auf Basis der Sequenz der angezeigten Selbstbilder und Fremdbilder durchgeführt. Dieses Verfahren kann vorteilhaft sein, weil das Verfahren keine vorausgehende Speicherung von sensiblen, biometrischen, personenbezogenen Daten beansprucht. Folglich ist das Verfahren auch in Situationen einsetzbar in denen eine Speicherung personen-bezogener, biometrischer Daten unerwünscht ist. Außerdem wird hierdurch die Privatsphäre der sich authentifizierenden Personen geschützt. Gegenüber anderen biometrischen Authentifizierungsverfahren, die aus dem Stand der Technik bekannt sind, ist das Verfahren zudem noch sicherer gegenüber einem Angreifer, denn biometrische Daten, die nicht gespeichert werden müssen, können nicht gestohlen werden.

Nach Ausführungsformen der Erfindung umfasst das Verfahren außerdem die Löschung der empfangenen Hirnaktivitätssignale nach dem Authentifizierungsvorgang. Beispielsweise kann die Löschung sämtliche ersten und zweiten Hirnaktivitätssignale und ggf. von diesen erstellten Kopien umfassen und z.B. von der Authentifizierungssoftware durchgeführt werden. Diese Ausführungsform der Erfindung kann vorteilhaft sein, weil dadurch die Privatsphäre der zu authentifizierenden Person geschützt wird. Hierdurch kann das Verfahren auch in Situationen eingesetzt werden bei denen eine Speicherung personenbezogener, biometrischer Daten unerwünscht ist. Außerdem ist das Verfahren gegenüber anderen biometrischen Authentifizierungsverfahren, die aus dem Stand der Technik bekannt sind, noch sicherer gegenüber einem Angreifer, denn biometrische Daten, die nicht gespeichert werden, können nicht gestohlen werden.

### Elektrische(r) Sensor(en)

Nach Ausführungsformen der Erfindung ist der KLHA-Sensor ein elektrischer Sensor. Insbesondere kann dieser Sensor dazu ausgebildet sein, Ladungsänderungen und/oder Ladungsumverteilungen in der Nähe des Sensors zu messen. Der Sensor kann z.B. als ein Sensor ausgebildet sein, der einen Instrumentenverstärker oder Differenzverstärker mit hoher Gleichtaktunterdrückung beinhaltet, wobei der Verstärker mit Messelektroden gekoppelt wird. Beispielsweise kann der elektrische Sensor in verschiedene Gegenstände oder Geräte integriert sein, die sich in der Nähe des Kopfes der zu authentifizierenden Person befinden. Beispielsweise kann der elektrische Sensor in einen Stuhl oder Sessel, insbesondere in die Kopfstütze dieses Stuhls bzw. Sessels, integriert sein. Es ist auch möglich, dass die Person sich im Zuge des Authentifizierungsprozesses in eine begehbare Box begibt und der elektrische Sensor in, unter oder auf einer Seitenwand oder Decke dieser Box integriert ist. Die Person kann also während des Authentifizierungsprozesses ihren Kopf zumindest innerhalb eines gewissen Radius frei bewegen und drehen, und es ist nicht erforderlich, dass die Person Elektroden an bestimmten Bereichen des Kopfes platziert, damit Hirnaktivitätssignale erfasst werden können.

Der elektrische Sensor kann zum Beispiel einen Hochpassfilter beinhalten oder an diesen gekoppelt sein der Signalkomponenten geringer Frequenz zurückhält. Beispielsweise kann der Hochpassfilter Hirnaktivitätssignale mit einer Frequenz von mindestens einem Herz ungehindert passieren lassen, aber Signale mit einer Frequenz von unter einem Herz abschwächen oder komplett herausfiltern. Hierdurch kann Hintergrundrauschen reduziert werden.

Der Verstärker kann es ermöglichen, Hirnaktivitätssignale mit einer Amplitude ab 10 Nanovolt zu erfassen. Ein elektrischer Sensor mit einer derartigen Signalverstärkung ist um ein Vielfaches sensitiver als konventionelle, kontaktbehaftete EEG Technologie, die in der Regel Hirnaktivitätssignale erst ab einer Amplitude von mindestens einigen Mikrovolt erkennen kann.

Gemäß Ausführungsformen der Erfindung beinhaltet die Authentifizierungssoftware ein Modul zur Datenvorverarbeitung , das dazu ausgebildet ist, Störsignale zu erkennen und durch automatische Filterung zu minimieren, sodass die ersten und zweiten Hirnaktivitätssignale als störungsminimierte, "entrauschte" erste und zweite Hirnaktivitätssignale bereitgestellt und von einem anderen Modul der Authentifizierungssoftware weiterverarbeitet werden. Die Störsignale können z.B. durch Vibrationen des KLHA-Sensors, durch den Herzschlag der einen Person, durch Ändern der Position und/oder Ausrichtung des Kopfes der einen Person im Raum und/oder durch Blinzelbewegungen oder starke Emotionen der einen Person hervorgerufen werden. Beispiele für geeignete Hochpassfilter und Signalverstärker sind in der internationalen Patentanmeldung WO 2017/189748 beschrieben.

Im Stand der Technik sind verschiedene Sensoren zur kontaktlosen Erfassung von Hirnaktivitätssignalen, insbesondere elektrische Sensoren, bekannt, die allerdings nicht zur Authentifizierung von Personen verwendet wurden.

Beispielsweise beschreibt die internationale Patentanmeldung WO 2017/189748 verschiedene Typen geeigneter Sensoren. Der elektrische Sensor kann beispielsweise in Form einer kontaktlosen Elektrode, zum Beispiel einer trockenen Elektrode, die kein Kontaktgel benötigt und die nicht in physischen Kontakt mit der einen Person ist, ausgebildet sein. Derartige Sensoren werden auch als "Biopotentialsensoren" bezeichnet. Die Größe und Form dieser Elektrode kann je nach Anwendungsszenario sehr unterschiedlich ausgebildet sein. Beispielsweise kann die Elektrode die Form mehrerer konzentrischer Ringe aufweisen, oder die Form mehrerer parallel zueinander angeordnete Stäbe. Vorzugsweise ist der elektrische Sensor als Array mehrerer Elektroden ausgebildet, sodass Hirnaktivitätssignale von mehreren Bereichen des Kopfes unterschiedliche Bereiche der Elektrode in unterschiedlicher Stärke erreichen. Dies erlaubt es dem elektrischen Sensor, zusätzliche Informationen bezüglich der Hirnregion, die ein Hirnaktivitätssignale erzeugt, zu erfassen. Beispielsweise kann dieser Array 5-6 Einzelelektroden beinhalten (eine im Vergleich zu 30-60 Kontaktelektroden bei einem konventionellen EEG immer noch geringe Zahl). Durch die Verwendung von elektrischen Sensoren mit mehreren Einzelelektroden bzw. einer räumlichen Ausdehnung von vorzugsweise mehr als 5 cm in zwei Dimensionen kann auch die Abhängigkeit der Signalstärke vom Abstand und Ausrichtung des Kopfes der zu authentifizierenden Person erfasst werden. Dies ermöglicht es zum Beispiel festzustellen, ob die zu authentifizierenden Person auch tatsächlich auf die Anzeigevorrichtung blickt, auf welcher aktuell die Fremdbilder bzw. Selbstbilder angezeigt werden (und nicht zum Beispiel auf ein heimlich mitgebrachtes Selbst-Foto, dessen Anblick es einem Maskenträger ermöglichen würde, Hirnaktivitätssignale, die typisch für die Betrachtung eines Selbstbilds sind, zu erzeugen, obwohl das offiziell gezeigte Selbstbild (Spiegelbild) nur die Maske zeigt.

Die Doktorarbeit "Kapazitive Elektroden zur Messung bioelektrischer Signale" von Martin Oehler, 2009, Institut für Elektrische Messtechnik und Grundlagen der Elektrotechnik, und dort insbesondere das Kapitel 3.2, beschreibt weitere Beispiele für elektrische Sensoren, die zur kontaktlosen Erfassung von Hirnstromsignalen (und Herzschlagsignalen) geeignet sind, sowie Beispiele dafür, wie die Verstärkung und der Hochpassfilter ausgebildet sein können. Ein elektrischer Sensor kann z.B. eine oder mehrere Elektroden umfassen und folgende Elemente beinhalten: eine oder mehrere Elektroden, wobei die Elektrodenfläche, die nach außen hin isoliert sein kann, das eigentliche Signal erfasst. Die Elektrode(n) ist/sind mit dem zweiten wichtigen Teil, der Elektronik, verbunden, welche das Signal hochimpedant aufnimmt und verstärkt. Das dritte Element des elektrischen Sensors ist die Abschirmung, auch "Filter" genannt, die sowohl aktiv als auch passiv ausgeführt sein kann.

Die Funktion der kapazitiven Elektrode basiert gemäß einer Ausführungsform der Erfindung auf einer extrem hochohmigen Messung des kapazitiv eingekoppelten Hirnaktivitätssignals ("Biosignals"). Die Elektrodenfläche und der Elektrodenkörper bilden einen Koppelkondensator. Die hochohmige Messung des Eingangssignals resultiert aus der Eingangscharakteristik, die sich aus dem Koppelkondensator und dem Eingangswiderstand der Schaltung ergibt. Dieser Eingang verhält sich wie ein RC-Hochpass für das eingekoppelte Signal.

Gemäß Ausführungsformen der Erfindung wird das Authentifizierungsverfahren innerhalb eines Authentifizierungssystems durchgeführt, wobei innerhalb des Authentifizierungssystems der zumindest eine KLHA-Sensor in räumlicher Nähe zum Kopf der zu authentifizierenden Person angebracht ist. Unter der räumlichen Nähe wird hier vorzugsweise ein Abstand von weniger als 100 cm, in manchen Ausführungsformen weniger als 70cm, in manchen Ausführungsformen von weniger als 30 cm, verstanden. Diese Abstände sind insbesondere beim elektrischen Sensor vorteilhaft, beim optischen Sensor kann der die räumliche Nähe definierende Abstand auch größer sein, z.B. mehrere Meter, sofern gewährleistet ist, dass die räumliche Auflösung des optischen Sensors eine bildanalysegestützte Erkennung von Hirnaktivitätssignalen wie z.B. Pupillendurchmesseränderungen zulässt.

Die von dem elektrischen Sensor erfassten Hirnaktivitätssignale können auch als Hirnstromsignale bezeichnet werden. Sie werden direkt durch elektrochemische Aktivitäten in den Zellen des Gehirns verursacht und geben Aufschluss über bewusste und/oder unbewusste Denkprozesse und Aktivitäten des Gehirns zum Zeitpunkt der Erfassung dieser Signale durch den Sensor.

Bei dem von einem elektrischen Sensor erfassten Hirnaktivitätssignale kann es sich insbesondere um Signale im Frequenzbereich von (von 0,1 bis zu 40 Hz) handeln.

Nach Ausführungsformen ist der elektrische Sensor dazu ausgebildet, die ersten und/oder zweiten Hirnaktivitätssignale durch Messung von Ladungsänderungen bzw. Ladungsumverteilungen in der Nähe des Sensors zu erfassen. Unter der Nähe wird hier vorzugsweise ein Bereich verstanden, der durch einen Abstand von dem elektrischen Sensor von weniger als 100 cm gekennzeichnet ist. Gemäß Ausführungsformen der Erfindung umfasst die räumliche Nähe einen Bereich mit einem Abstand von 1 cm bis 100 cm von dem Sensor, in manchen Ausführungsformen von 1 cm bis 70 cm vom Sensor, in anderen Ausführungsformen von 1 cm bis 60 cm. **In** manchen Fällen wird der Sensor so positioniert, dass er sich in einem Abstand von 15-60 cm vom Kopf der Person entfernt befindet.

Aus dem erfassten Signal können die Amplitude und/oder der zeitliche Amplitudenverlauf der ersten und/oder zweiten Hirnaktivitätssignale ermittelt werden.

Gemäß einer Ausführungsform umfasst die Analyse der erfassten Hirnaktivitätssignale einen Vergleich von Amplitudenverlaufsprofilen der ersten Hirnaktivitätssignale und von Amplitudenverlaufsprofilen der zweiten Hirnaktivitätssignale untereinander, um festzustellen, ob sich die ersten Hirnaktivitätssignale signifikant von den zweiten Hirnaktivitätssignalen unterscheiden.

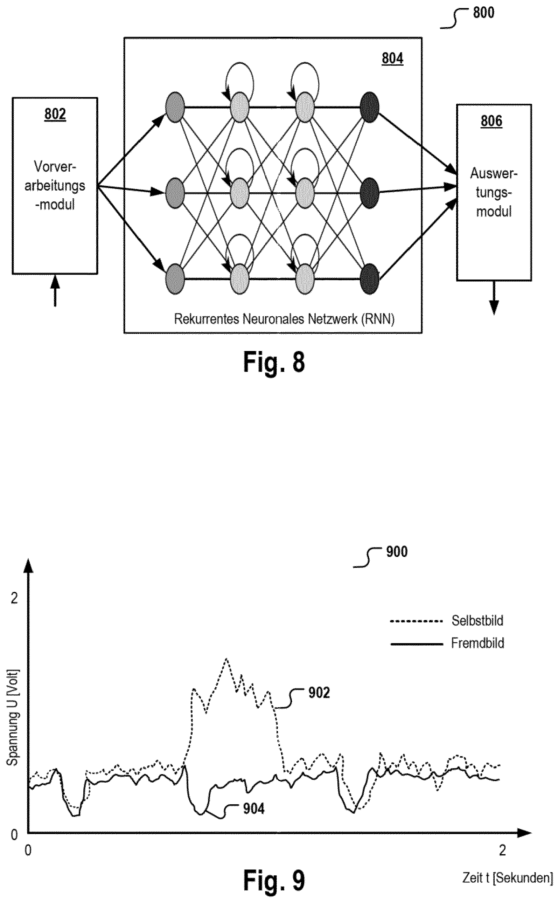

Nach Ausführungsformen sind die ersten Hirnaktivitätssignale identisch oder ähnlich zu den Hirnaktivitätssignalen gemäß Kurve 902 in Figur 9. Zusätzlich oder alternativ dazu sind die zweiten Hirnaktivitätssignale identisch oder ähnlich zu den Hirnaktivitätssignalen gemäß Kurve 904 in Figur 9. Das bedeutet, dass bei vielen Menschen innerhalb eines Zeitraums von ca. 500 ms bis 1000 ms nach dem Beginn des Betrachtens eines Selbstbildes eine deutliche Verstärkung des Signals (Amplitudenzuwachs um ca. 100% relativ zum Ausgangszustand) beobachtbar ist, wohingegen beim Betrachten eines Fremdbildes dieser Anstieg ausbleibt oder sich die Signalstärke im Zeitraum von ca. 500-700 ms nach dem Beginn des Betrachtens des Fremdbildes sogar verringert. Die Analyse der erfassten Hirnaktivitätssignale umfasst einen Vergleich von Amplitude und/oder Amplitudenverlauf der ersten Hirnaktivitätssignale und zweiten Hirnaktivitätssignale untereinander, um festzustellen, ob sich die ersten Hirnaktivitätssignale signifikant von den zweiten Hirnaktivitätssignalen unterscheiden.

Gemäß bevorzugten Ausführungsformen umfasst die Analyse lediglich einen Vergleich der Amplituden und zeitlichen Amplitudenprofile der ersten Hirnaktivitätssignalen und zweiten Hirnaktivitätssignalen jeweils untereinander und mit dem anderen Hirnaktivitätssignaltyp unter Berücksichtigung der bekannten zeitlichen Abfolge der Anzeige der ein oder mehreren Selbstbilder und der ein oder mehreren Fremdbilder. Hirnaktivitätssignale, die während der Anzeige eines Selbstbilds erfasst werden, werden als erste Hirnaktivitätssignale erfasst. Hirnaktivitätssignale, die während der Anzeige eines Fremdbilds erfasst werden, werden als zweite Hirnaktivitätssignale erfasst. Wenn die Person wirklich die Person ist, die auf dem angezeigten Selbstbild abgebildet ist, ist also zu erwarten, dass alle erfassten ersten Hirnaktivitätssignale identische oder sehr ähnliche Eigenschaften (Amplitude, Amplitudenverlauf über die Zeit, ggf. weitere Eigenschaften des Signals wie z.B. Frequenz) haben. Ebenso ist zu erwarten, dass die erfassten zweiten Hirnaktivitätssignale identische oder zumindest ähnliche Eigenschaften haben, wobei sich allerdings die Eigenschaften der ersten und der zweiten Hirnaktivitätssignale signifikant unterscheiden. Trägt die zu authentifizierenden Person dagegen eine Maske oder ist das angezeigte Bild ein "gemorphtes" Bild, dann reagiert das Hirn der zu authentifizierenden Person so, als ob ein Fremdbild angezeigt würde. In diesem Fall haben die ersten Hirnaktivitätssignale identische oder ähnliche Eigenschaften wie die zweiten Hirnaktivitätssignale. In diesem Fall behandelt die Authentifizierungssoftware die Authentifizierung der einen Person als gescheitert.

### Optischer Sensor

Nach Ausführungsformen ist der KLHA-Sensor ein optischer Sensor, insbesondere eine Kamera oder eine Videokamera. Bei dem optischen Sensor handelt es sich insbesondere um eine IR-Kamera oder eine IR-Videokamera. Gemäß manchen Ausführungsformen kann es sich bei dem optischen Sensor jedoch auch um eine Kamera oder Videokamera handeln, die zur Erfassung von Bildern oder Videos im Wellenlängenbereich des sichtbaren Lichts ausgebildet ist. Bei diesen Ausführungsformen ist der KLHA-Sensor ein Sensor, welcher ein oder mehrere Signale empfangen kann, die von einem Menschen ausgesendet werden, ohne dass während des Empfangs der Signale ein Kontakt des Sensors zu dem Menschen besteht, wobei die empfangenen Signale direkt oder indirekt Ausschluss über Hirnaktivitäten des Menschen geben. Die ersten und zweiten Hirnaktivitätssignale sind von der einen Person ausgestrahlte Signale, welche durch eine Hirnaktivität direkt oder indirekt bewirkt werden.

Nach Ausführungsformen ist der KLHA-Sensor dazu ausgebildet, Bilder und/oder Bildsequenzen des Gesichts oder von Teilen des Gesichts der einen Person zu erfassen. Aus den Bildern, die beim Betrachten von Fremdbildern und Selbstbildern erfasst wurden, können absolute Pupillendurchmesser und damit auch Unterschiede im Pupillendurchmesser bei der Betrachtung von Bildern unterschiedlichen Typs ermittelt werden. Aus den Bildsequenzen wird der zeitliche Verlauf des Pupillendurchmessers beim Betrachten eines bestimmten Bildes ermittelt. Aus dem ermittelten Signal können die Amplitude (Pupillendurchmesser) und/oder der zeitliche Amplitudenverlauf der ersten und/oder zweiten Hirnaktivitätssignale ermittelt werden. Ein "Amplitudenverlauf" oder "Amplitudenverlauf über die Zeit" wird hier auch als "Profil" oder "Amplitudenprofil" bezeichnet.

Gemäß Ausführungsformen umfasst das Verfahren eine automatische Bildanalyse der Bilder oder Bildsequenzen, um einen Pupillendurchmesser der einen in dem Bild abgebildeten Person zu bestimmen. Die Bestimmung des Pupillendurchmessers kann durch die Authentifizierungssoftware oder durch ein anderes Softwareprogramm durchgeführt werden.

Beispielsweise umfasst die Bildanalyse eine Messung (im Sinne einer rechnerischen Bestimmung anhand der Bilddaten) eines Pupillendurchmessers der einen Person. Zusätzlich oder alternativ dazu umfasst die Bildanalyse eine Messung (im Sinne einer rechnerischen Bestimmung anhand der Bilddaten) eines Pupillendurchmesseränderungsprofils der einen Person (also die Änderung dieses Durchmessers über die Zeit).

Gemäß einer Ausführungsform umfasst die Analyse der erfassten Hirnaktivitätssignale einen Vergleich von Amplitudenverlaufsprofilen der ersten Hirnaktivitätssignale und von Amplitudenverlaufsprofilen der zweiten Hirnaktivitätssignale untereinander, um festzustellen, ob sich die ersten Hirnaktivitätssignale signifikant von den zweiten Hirnaktivitätssignalen unterscheiden.

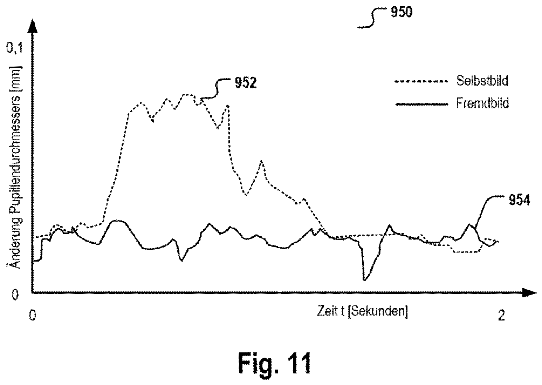

Nach Ausführungsformen sind die ersten Hirnaktivitätssignale identisch oder ähnlich zu den Hirnaktivitätssignalen gemäß Kurve 952 in Figur 11. Nach Ausführungsformen sind die die zweiten Hirnaktivitätssignale identisch oder ähnlich zu den Hirnaktivitätssignalen gemäß Kurve 954 in Figur 11. Das bedeutet, dass bei vielen Menschen innerhalb der ersten 1500 ms nach dem Beginn des Betrachtens eines Selbstbildes eine deutliche Vergrößerung des Pupillendurchmessers (um ca. 0,05 mm - 0,50 mm) beobachtbar ist, die beim Betrachten eines Fremdbildes ausbleibt.

Nach Ausführungsformen umfasst die Analyse der erfassten Hirnaktivitätssignale die Durchführung eines Bildverarbeitungsprozesses. Dabei werden unter Zuhilfenahme von Verfahren des Machine Learnings und Deep Learnings Algorithmen trainiert, die selbstständig Unterschiede bezüglich des Pupillendurchmessers während der Betrachtung des Fremd- und Selbstbilder detektieren und so eine Klassifizierung vornehmen können.

Beispielsweise kann es sich bei dem optischen Sensor um eine Kamera oder Videokamera handeln, die im Infrarotbereich oder im Wellenlängenbereich des sichtbaren Lichtes ein oder mehrere Bilder des Gesichts oder von Teilen des Gesichts der zu authentifizierenden Person aufnimmt und an die Authentifizierungssoftware weiterleitet. Die Authentifizierungssoftware führt eine Bildanalyse durch, beispielsweise um einzelne Komponenten der Augenpartie des Gesichtes zu erkennen, insbesondere Iris und/oder Pupille von einem oder beiden Augen der zu authentifizierenden Person. Durch Analyse der Position und/oder Größe der Pupille einer Person in mehreren über die Zeit aufgenommenen Bildern kann außerdem ein Pupillendurchmesseränderungsprofil über ein Zeitintervall (von z.B. 0-4 Sekunden Dauer nach dem Beginn des Anzeigens eines Selbstbilds oder Fremdbilds, insb. von 0-4 Sekunden Dauer) von einem oder beiden Augen dieser Person durch Bildanalyse ermittelt werden.

Die Anmelderin hat festgestellt, dass der Pupillendurchmesser und die Änderung des Pupillendurchmessers über die Zeit Ausdruck von Hirnaktivitäten sind, und insbesondere auch signifikant unterschiedlich sind in Abhängigkeit davon, ob einer Person ein Selbstbild oder ein Fremdbild angezeigt wird. Somit ist es möglich, durch automatisches Erfassen und Analysieren von Bildern der Augenpartie von Personen während eines Zeitraums, innerhalb welchem der Person ein oder mehrere Selbstbilder und ein oder mehrere Fremdbilder gezeigt werden, zu erkennen, ob sich der oder die Pupillendurchmesser, die während des Betrachtens eines Selbstbildes gemessen wurden, signifikant von dem oder den Pupillendurchmessern, die während des Betrachtens eines Fremdbilds gemessen werden, unterscheiden, wohingegen die entsprechenden Signale, die beim Betrachtens des gleichen Bildtyps (Selbstbild oder Fremdbild) gemessen werden, identisch oder sehr ähnlich sind. Somit eignen sich die besagten biometrische Merkmale "Pupillendurchmesser" bzw. "Pupillendurchmesseränderungsprofil" dazu, die Person auf Basis des Authentifizierungsverfahrens gemäß Ausführungsformen der Erfindung zu authentifizieren.

Die oben genannten biometrischen Merkmale geben zumindest indirekt Aufschluss über Hirnaktivitäten, denn auch wenn bei diesen Merkmalen die Hirnaktivität nicht direkt in Form von Hirnstromsignalen gemessen wird, so werden doch physiologische Parameter erfasst, die auf direkte und bewusst nicht steuerbare Weise von den Hirnaktivitäten abhängen, die durch das Betrachten von Gesichtsbildern in der Person ausgelöst werden. Hierbei ist hervorzuheben, dass nicht jegliche Art physiologischer Parameter zur Authentifizierung der Person geeignet ist, denn wenn diese physiologische Parameter nicht einerseits zumindest bei der einen Person bei Bildern des gleichen Typs (entweder Selbstbild oder Fremdbild) reproduzierbar identische oder sehr ähnliche Eigenschaften aufweist und gleichzeitig die Signale beim Betrachten von Bildern unterschiedlichen Typs signifikant unterschiedliche Eigenschaften aufweisen, sind sie nicht geeignet, als Basis für das Authentifizierungsverfahren Verwendung zu finden.

Die Anmelderin hat beobachtet, dass eine deutliche Vergrößerung des Pupillendurchmessers innerhalb der ersten 2000 ms, vorzugsweise innerhalb der ersten 1000 ms nach dem Anzeigen eines Bildes (z.B. eine Vergrößerung von mindestens 8%, vorzugsweise mindestens 15%), ein Indiz dafür ist, dass der Betrachter sich selbst im Bild erkennt, dass das gezeigte Bild also ein Selbstbild ist. Bleibt diese Pupillenweitung aus, handelt es sich um ein Fremdbild.

Gemäß einer Ausführungsform wird eine Bestimmung der Augenlidöffnung mehrfach pro angezeigtem Bild gemessen und dadurch ein oder mehrere Blinzelbewegungen der einen Person erfasst. Vorzugsweise dienen die erfasste Blinzelbewegungen dazu, ein erfasstes Hirnaktivitätssignal zu korrigieren. Dies kann vorteilhaft sein, da das Blinzeln in manchen Fällen auch eine Störung anderer Hirnaktivitätssignale wie z.B. der von einem elektrischen Sensor erfassten Hirnstromsignale bewirkt. Durch eine entsprechende Korrektur wird die Akkuratheit der Vorhersage erhöht.

Gemäß einer Ausführungsform werden die Fremdbilder so gewählt oder deren Helligkeit so normalisiert, dass diese einen ähnlichen Helligkeitsgrad aufweisen wie das eine Bekanntbild, oder wie ein mittlerer Helligkeitsgrad mehrerer Bekanntbilder oder wie der Helligkeitsgrad des zuletzt gezeigten Bekanntbildes. Beispielswiese kann ein ähnlicher Helligkeitsgrad bedeuten, dass der Mittelwert aller Pixelintensitäten des Fremdbildes nicht mehr als 25%, vorzugweise nicht mehr als 10% unter oder über dem Mittelwert aller Pixelintensitäten des Bekanntbildes oder der vorgenannten Bekanntbilder liegt.

Dies kann den Vorteil haben, dass Änderungen des Pupillendurchmessers, die auf die Adaptation an unterschiedliche Helligkeitsverhältnisse und nicht auf die Fremdbild/Bekanntbild-Rezeption zurückzuführen sind und die als Störsignale zu betrachten sind, reduziert oder vermieden werden.

Nach Ausführungsformen der Erfindung umfasst das Verfahren Machine-Learning Verfahrensschritte, z.B. um im Zuge einer Trainingsphase einen personenspezifischen oder generischen Klassifikator zu erzeugen, der dazu ausgebildet ist, die Unterschiede der ersten und/oder zweiten Hirnaktivitätssignale zu lernen (z.B. während der Registrierung eines Nutzers), und/oder im Zuge einer Test- bzw. Anwendungsphase des trainierten Klassifikators zur Unterscheidung der ersten und/oder zweiten Hirnaktivitätssignale während eines Authentifizierungsprozesses.

Nach einer Ausführungsform wird die Bildanalyse von einem neuronalen Netz oder einer anderen Machine-Learning- Software (zum Beispiel Supportvektormaschine) durchgeführt. Die Machine-Learning- Software kann beispielsweise in einer Trainingsphase gelernt haben, bestimmte Gesichtsregionen bzw. Augenregionen wie zum Beispiel Iris und/oder Pupille automatisch zu erkennen und optional auch Größe und/oder Position von Pupille (und/oder ggf. für Signalkorrekturzwecke auch der Augenlidöffnung) zu bestimmen. Das Ergebnis der Bildanalyse besteht entsprechend aus ein oder mehreren numerischen Werten, die die Pupillengröße und/oder ein Pupillengrößenänderungsprofil über ein Zeitintervall spezifizieren. Zumindest ein oder mehrere dieser numerischen Werte werden als Eingabe an ein weiteres Softwaremodul der Authentifizierungssoftware weitergegeben. Das weitere Modul ist dazu ausgebildet, derartige numerische Werte, die jeweils beim Betrachten von Selbstbildern erfasst wurden, als erste Hirnaktivitätssignale zu verarbeiten, und entsprechende numerische Werte, die jeweils beim Betrachten von Fremdbildern erfasst wurden, als zweite Hirnaktivitätssignale zu verarbeiten. Im nächsten Schritt ermittelt die Authentifizierungssoftware die Ähnlichkeit der ersten Hirnaktivitätssignale untereinander (also die Ähnlichkeit mehrerer erster Hirnaktivitätssignale die beim Betrachten mehrerer Selbstbilder erfasst werden untereinander), die Ähnlichkeit der zweiten Hirnaktivitätssignale untereinander (also die Ähnlichkeit mehrerer zweiten Hirnaktivitätssignale die beim Betrachten mehrerer Fremdbilder erfasst werden untereinander) sowie die Ähnlichkeit der ersten Hirnaktivitätssignale und der zweiten Hirnaktivitätssignale. Für den letztgenannten Vergleich können verschiedene Verfahren verwendet werden, beispielsweise Clusteranalysen oder ein Mehrschrittverfahren, in welchem ein erster Mittelwert aus allen ersten Hirnaktivitätssignalen berechnet wird, ein zweiter Mittelwert aus allen zweiten Hirnaktivitätssignalen berechnet wird, und sodann die Ähnlichkeit des ersten und zweiten Mittelwerts berechnet wird. Durch Vergleich der berechneten Ähnlichkeiten bzw. Abweichungen mit vordefinierten Grenzwerten kann die Authentifizierungssoftware automatisch feststellen, ob die ersten und zweiten Hirnaktivitätssignale signifikant voneinander unterschieden sind.

Nach Ausführungsformen umfasst die Analyse der erfassten Hirnaktivitätssignale die Ausführung eines Machine-Learning-Programms.

Beispielsweise kann ein "supervised learning" Ansatz dazu verwendet werden, zeitliche Profile der Signalamplituden (z.B. Spannung eines elektrischen Feldes im Falle von elektrischen Sensoren, Pupillendurchmesser im Falle von optischen Sensoren), die von einer Person je beim Betrachten von Fremdbildern oder Selbstbildern erzeugt werden, zu analysieren und zu lernen, auf Basis dieser Verläufe den Typ des betrachteten Bildes automatisch zu erkennen bzw. vorherzusagen. Beispielsweise kann es sich bei dem Machine-Learning-Programm um ein neuronales Netzwerk oder eine Supportvektormaschine handeln. Insbesondere kann das neuronale Netz als ein rekurrentes neuronales Netzwerk ausgebildet sein. Das neuronale Netz lernt während des Trainings auf Grundlage von zeitlichen Verläufen der Signalamplitude (z.B. Durchmesser Pupille in Micrometer), wie die Signalamplituden bzw. Signalamplitudenprofile bei der Betrachtung von Selbstbildern und Fremdbildern jeweils aussehen und/oder wie diese sich bei der Betrachtung von Selbstbildern und Fremdbildern unterscheiden.

Das neuronale Netzwerk kann beispielsweise auf Trainingsdaten, die zu einer einzigen Person oder zu mehreren verschiedenen Personen gehören, trainiert werden.

Gemäß Ausführungsformen wird das vom elektrischen Sensor erfasste Rohsignal als erstes oder zweites Hirnaktivitätssignal verwendet und ausgewertet. Bei dem Rohsignal kann es sich um die Gesamtheit der innerhalb eines oder mehrerer Frequenzbereiche erfassten Hirnaktivitätssignale handeln oder um eine oder mehrere diskrete Frequenzen. Gemäß anderer Ausführungsformen wird das vom elektrischen Sensor erfasste Rohsignal zunächst vorverarbeitet, z.B. normalisiert, entrauscht, und/oder gefiltert, um die Signalqualität und/oder Vergleichbarkeit des Signals zu erhöhen, und dieses vorverarbeitete Hirnaktivitätssignal wird als erstes oder zweites Hirnaktivitätssignal verwendet und ausgewertet.

Eine über die Bestimmung des Pupillendurchmessers hinausgehende Verarbeitung von Bilddaten ist nicht notwendig, da keine Objektklassifierzung stattfindet. Das Training kann daher auf Grundlage von annotierten Daten (z.B. Pupillendurchmesseran oder Pupillendurchmesseränderungsprofilen, die mit "bei Selbstbildbetrachtung" oder "bei Fremdbildbetrachtung" annotiert sind, performant durchgeführt werden.

Die für das Training verwendeten annotierten Trainingsdaten werden anhand eines Protokolls aufgezeichnet und annotiert, damit das Modell trainiert und verifiziert werden kann.

Nach Ausführungsformen umfasst die Analyse der erfassten Hirnaktivitätssignale die Erkennung einer Korrelation von signifikant unterschiedlichen Hirnaktivitätssignalen zu Bildtypwechselzeitpunkten, wobei an einem Bildtypwechselzeitpunkt ein in der Anzeige gezeigtes Selbstbild durch ein Fremdbild ersetzt wird oder umgekehrt.

In einem weiteren Aspekt betrifft die Erfindung ein Authentifizierungssystem zur Authentifizierung einer Person, das dazu konfiguriert ist, das Authentifizierungsverfahren gemäß einer der hier beschriebenen Ausführungsformen durchzuführen.

In einem weiteren Aspekt betrifft die Erfindung ein Authentifizierungssystem zur Authentifizierung einer Person. Das Authentifizierungssystem umfasst eine Anzeigevorrichtung, eine Authentifizierungssoftware und zumindest einen kontaktlosen Sensor zur Sensierung von Hirnaktivitäten. Der zumindest eine Sensor wird im Folgenden als KLHA-Sensor bezeichnet. Die Authentifizierungssoftware ist konfiguriert zur Anzeige mehrerer Gesichtsbilder, die Gesichter mehrerer unterschiedlicher Personen abbilden, auf der Anzeige für die eine Person. Die mehreren Gesichtsbilder umfassen ein der mehrere Selbstbilder. Ein Selbstbild ist ein Gesichtsbild dieser einen Person. Der KLHA-Sensor ist dazu konfiguriert, während die mehreren Gesichtsbilder der einen Person angezeigt werden, erste und zweite Hirnaktivitätssignale, die in der Person durch die Betrachtung der Gesichtsbilder jeweils ausgelöst werden, zu erfassen. Die ersten Hirnaktivitätssignale werden erfasst, während dieser einen Person die ein oder mehreren Selbstbilder angezeigt werden. Die zweiten Hirnaktivitätssignale werden erfasst, während dieser einen Person die ein oder mehreren Fremdbilder angezeigt werden. Die Authentifizierungssoftware ist ferner konfiguriert ist zur Analyse der erfassten ersten und zweiten Hirnaktivitätssignale, um die eine Person gegenüber der Authentifizierungssoftware zu authentifizieren. Die eine Person wird nur dann als erfolgreich authentifiziert behandelt, wenn die ersten Hirnaktivitätssignale sich signifikant unterscheiden von den zweiten Hirnaktivitätssignalen.

Nach Ausführungsformen ist der KLHA-Sensor ein elektrischer Sensor zur Erfassung eines Hirnsignals ohne direkten Kontakt des Kopfes mit dem elektrischen Sensor. Insbesondere kann der elektrische Sensor als isolierte kapazitive Elektrode ausgebildet sein, wobei der elektrische Sensor vorzugsweise einen Signalverstärker umfasst.

Nach anderen Ausführungsformen ist der KLHA-Sensor ein optischer Sensor, z.B. eine IR-Kamera oder IR-Videokamera.

Nach Ausführungsformen umfasst das Authentifizierungssystem ein Terminal, das die Anzeige beinhaltet. Bei dem Terminal kann es sich insbesondere um ein Flughafenterminal, ein Grenzkontrollterminal, und/oder ein Terminal zur Kontrolle des Zutritts zu einem geschützten geographischen Bereich handeln.

Gemäß einer Ausführungsform wird, z.B. im Zuge der Registrierung einer Person bei der authentifizierenden Instanz, durch Messung von Hirnaktivitätssignalen während der Betrachtung mehrerer Bekanntbilder und Fremdbilder ein Trainingsdatensatz erzeugt und ein personen-individueller Klassifikator auf diesen Bildern trainiert. Der trainierte Klassifikator ist in der Lage, Hirnaktivitätssignale speziell dieser Person in solche zu klassifizieren, die das Resultat der Betrachtung eines Bekanntbildes sind und in solche, die das Resultat einer Betrachtung eines Fremdbildes sind. Der personenspezifisch trainierte Klassifikator kann z.B. als Bestandteil des Nutzerprofils dieser Person gespeichert werden.

In einer alternativen Ausführungsform wird ein vereinfachter Registrierungsprozess (Enrollment) und ein vereinfachter Authentisierungsprozess durchgeführt. In dieser Ausführungsform wird nicht ein individuell auf den Hirnaktivitätssignalen einer bestimmten Person trainierter Klassifikator verwendet, um festzustellen, ob die Eigenschaften der erfassten Hirnaktivitätssignale zu den Eigenschaften der Hirnaktivitätssignale passen, wie diese angesichts der Sequenz an angezeigten Bekannt- und Fremdbildern zu erwarten wären. Vielmehr wird ein generischer Klassifikator, der nicht individuell für eine bestimmte Person angelernt wurde, sondern auf der Basis von Hirnaktivitätssignalen einer Vielzahl von Personen erstellt wurde, verwendet. In dem Fall müsste während der Registrierung lediglich ein verifiziertes Bild, z.B. das elektronische Passbild vom neuen Personalausweis, an das System übermittelt werden.

Mehrere Ausführungsformen der Erfindung wurden unter Bezugnahme auf eine Behörde als Beispiel für eine authentifizierende Instanz beschrieben. Anstelle einer Behörde kann jedoch auch eine private Organisation, z.B. ein Unternehmen, ein Verein oder eine sonstige Entität, gegenüber welcher sich ein Nutzer authentifizieren möchte, fungieren.

Unter einer **"Anzeigevorrichtung"** wird hier ein Gerät verstanden, das dazu ausgebildet ist, zeitlich veränderlichen Informationen wie Bilder oder Zeichen anzuzeigen. Insbesondere kann es sich bei der Anzeigevorrichtung um einen Bildschirm oder sonstige Form einer elektrisch angesteuerten Anzeige handeln. Es kann sich aber auch um eine Kombination eines Projektors mit einer Projektionsfläche handeln. Die Anzeigevorrichtung kann dabei ein eigenständiges Gerät oder Teil eines Gerätes sein, z.B. Teil eines Terminals.

Unter einem "Sensor", auch als Detektor oder (Mess-)Fühler bezeichnet, ist ein technisches Bauteil oder Gerät, das bestimmte physikalische und/oder chemische Eigenschaften (insbesondere Impedanz, elektrische Felder, elektromagnetische Felder, optische Signale, Ladungsänderungen und/oder Ladungsumverteilungen in der Umgebung, etc.) qualitativ oder quantitativ erfassen kann. Diese Größen werden mittels physikalischer oder chemischer Effekte erfasst und vom Sensor in ein weiterverarbeitbares elektrisches Signal umgeformt.

Unter einem **"optischen Sensor"** wird hier ein Sensor bezeichnet, welcher dazu ausgebildet ist, ein oder mehrere Bilder eines physischen Objekts, z.B. eines Gesichts oder Gesichtsbereichs, zu erfassen. Die Bilder können insbesondere digitale Bilder sein. Der optische Sensor kann z.B. eine Kamera oder eine Videokamera sein. Der optische Sensor kann z.B. dazu ausgebildet sein, selektiv Licht eines bestimmten Wellenlängenbereichs zu erfassen, z.B. Infrarotlicht (IR-Licht) oder Licht im für das menschliche Auge sichtbaren Wellenlängenbereich (Weißlicht, Tageslicht). Die erfassten Bilder werden chemisch oder elektronisch aufgezeichnet. Vorzugsweise handelt es sich bei den erfassten Bildern um digitale Bilder, die elektronisch gespeichert und/oder direkt an eine Authentifizierungssoftware bereitgestellt werden.

Unter einem **"elektrischen Sensor"** wird hier ein Sensor verstanden, der dazu ausgebildet ist, Ladungsänderungen und/oder Ladungsumverteilungen in seiner Umgebung zu messen, wobei die Umgebung der räumliche Sensitivitätsbereich des Sensors ist.

Unter einem **"kontaktlosen Hirnaktivitäts-Sensor (KLHA-Sensor)"** wird hier ein Sensor verstanden, welcher ein oder mehrere Signale empfangen kann, die von einem Menschen ausgesendet werden, ohne dass während des Empfangs der Messwerte ein Kontakt des Sensors zu dem Menschen besteht. Die empfangenen Messwerte geben direkt (Hirnstromsignale gemessen durch einen kontaktlosen elektrischen Sensor) oder indirekt (Pupillendurchmesser, Pupillendurchmesseränderungsprofil) Aufschluss über Hirnaktivitäten des Menschen, von dem die Signale empfangen werden, zum Zeitpunkt des Empfangs.

Unter einer **"Hirnaktivität"** wird hier die physiologische Aktivität einer Vielzahl von Neuronen des Gehirns eines Menschen verstanden. Die physiologische Aktivität korrespondiert in elektrischen Zustandsänderungen der Neuronen zur Informationsverarbeitung des Gehirns.

Unter einem **"Hirnaktivitätssignal"** wird hier ein von einem Menschen ausgestrahltes Signal verstanden, welches durch eine Hirnaktivität direkt oder indirekt bewirkt wird. Beispielsweise addieren sich die über den gesamten Kopf verteilten elektrischen Zustandsänderungen und Potentialänderungen der Neuronen gemäß ihrer spezifischen räumlichen Anordnung auf und lassen sich direkt als sogenannte "Hirnstromsignale" mittels eines elektrischen Sensors messen. Bei einem Hirnaktivitätssignal kann es sich aber auch um jegliches andere durch einen physiologischen Prozess in einem menschlichen Körper emittierte Signal handeln, welches von einer Hirnaktivität hervorgerufen wird und sich vorzugsweise bewusst nicht oder nur eingeschränkt kontrollieren lässt. Beispielsweise ist das Einstellen der Iris auf einen bestimmten Pupillendurchmesser abhängig von unbewussten neuronalen Verarbeitungsprozessen im menschlichen Gehirn, die z.B. davon abhängen, was eine Person gerade sieht, z.B. ein Bild ihrer selbst oder ein Bild einer anderen Person (Fremdbild).

Unter einem **"ersten Hirnaktivitätssignal"** wird hier ein Hirnaktivitätssignal verstanden, welches erfasst wird, während eine Person ein Selbstbild betrachtet.

Unter einem **"zweiten Hirnaktivitätssignal"** wird hier wird hier ein Hirnaktivitätssignal verstanden, welches erfasst wird, während eine Person ein Fremdbild betrachtet

Unter einem **"Hirnstromsignal"** wird hier ein Hirnaktivitätssignal verstanden, welches sich ergibt durch Überlagerung einer Vielzahl von über den gesamten Kopf einer Person verteilten elektrischen Zustandsänderungen, insb. Potentialänderungen, der Neuronen gemäß ihrer spezifischen räumlichen Anordnung.

Unter einem **"Gesichtsbild"** wird hier ein Bild, insbesondere ein digitales Bild, verstanden, welches das Gesichts oder einen Gesichtsbereich einer Person abbildet.

Unter einem **"Selbstbild"** wird hier ein Bild, insbesondere ein Gesichtsbild, verstanden, das das Gesicht oder den Gesichtsbereich derjenigen Person abbildet, die sich gegenüber der Authentifizierungssoftware authentifizieren möchte.

Unter einem **"Fremdbild"** wird hier ein Bild, insbesondere ein Gesichtsbild, verstanden, das das Gesicht oder den Gesichtsbereich einer Person abbildet, die nicht diejenige Person (und auch kein eineiiger Zwilling dieser Person) ist, die sich gegenüber der Authentifizierungssoftware authentifizieren möchte.

Ein **"digitales Bild"** ist ein Datensatz, in dem Bildinhalte repräsentiert und gespeichert werden. Insbesondere kann es sich bei dem digitalen Bild um einen Datensatz handeln, in dem der Inhalt eines Bildes durch ganze Zahlen repräsentiert wird. Insbesondere kann es sich bei dem digitalen Bild um eine Rastergraphik handeln.

Unter einer **"Challenge"** wird hier eine Menge aus einem oder mehreren Datenwerten verstanden, welche einer ersten Partei bekannt ist, und welche einer zweiten Partei auf eine bestimmte Weise übermittelt wird um es der zweiten Partei zu ermöglichen, sich durch Nachweis der Kenntnis der Challenge oder durch Nachweis einer vordefinierten Verarbeitung der Challenge bei der ersten Partei zu authentifizieren.

Unter einer **"signifikanten Ähnlichkeit"** von Datenwerten wie zum Beispiel Hirnaktivitätssignalen wird hier insbesondere eine Situation verstanden, in welchem zwei Signale als im Rahmen der Messgenauigkeit identisch oder sehr ähnlich angesehen werden. Unter Berücksichtigung der Messungenauigkeit des Sensortyps und Sensormodells können "signifikant ähnliche Signale" also Signale sein, deren Unterschiedlichkeits-Score unterhalb eines vordefinierten Grenzwerts liegt, wobei der Grenzwert so gewählt ist, dass Unterschiedlichkeits-Scores von Hirnaktivitätssignalen, die von im Wesentlichen identischen oder sehr ähnlichen Vorgängen im Kopf der Person erzeugt werden, unterhalb dieses Grenzwerts liegen.

Unter einer **"signifikanten Unähnlichkeit"** von Datenwerten wie zum Beispiel Hirnaktivitätssignalen wird hier verstanden, dass die verglichenen Daten sich so stark voneinander unterscheiden, dass es sehr unwahrscheinlich ist, dass die Unterschiede auf messtechnische oder sonstige Varianzen (Messfehler) zurückzuführen sind. Bei der messtechnischen Erfassung von Signalen (z.B. mit optischem oder elektrischem Sensor) ist es unvermeidlich, dass hierbei leichte Schwankungen der Signalstärke auftreten. Die hierdurch bedingte Varianz, auch Messfehler oder "Hintergrundrauschen" bezeichnet, hat je nach Sensortyp und Sensormodell eine unterschiedliche Größe und Beschaffenheit, sodass es nicht möglich ist, für alle denkbaren Typen und Modelle von Sensoren eine absolute Grenze vorzugeben, ab welcher Höhe Signalunterschiede noch im Rahmen der Messungenauigkeit unterschiedlich sind und wann sie dagegen "signifikant unterschiedlich" ("signifikant unähnlich") sind, also so unterschiedlich sind, dass die Unterschiede nicht mehr auf die übliche Messungenauigkeit zurückführbar sind. Dem Fachmann ist der Umgang mit Messungenauigkeiten beim Verarbeiten und Vergleichen von gemessenen Signalen jedoch bekannt. Durch Wahl geeigneter Grenzwerte, z.B. eines Mindestabstands zwischen zwei Signalamplituden oder einer Mindestfläche, die sich errechnet als Differenz der Flächen unter zwei Amplitudenprofile der beiden Signale über die Zeit, kann der Fachmann definieren, ab welcher Unterschiedlichkeitsschwelle zwei Hirnaktivitätssignale als "signifikant unterschiedlich" gelten und wann die Signale dagegen als "identisch oder ähnlich im Rahmen der jeweiligen Messgenauigkeit" gelten.

Unter Berücksichtigung der Messungenauigkeit des Sensortyps und Sensormodells können "signifikant unähnliche Signale" oder "Signale, die signifikant unterschiedlich sind" also Signale sein, deren Unterschiedlichkeits-Score über einem vordefinierten Grenzwert liegt, wobei der Grenzwert so gewählt ist, dass Unterschiedlichkeits-Scores von Hirnaktivitätssignalen, die von im Wesentlichen unterschiedlichen Vorgängen im Kopf der Person erzeugt werden (z.B. beim Betrachten von Fremdbildern einerseits und von Selbstbildern andererseits), über diesem Grenzwert liegen.

Unter einer **"Authentifizierungssoftware"** wird hier Software verstanden, die dazu ausgebildet ist, ein Authentifizierungsverfahren durchzuführen, um eine bestimmte Person individuell oder als Mitglied einer Gruppe zu identifizieren. Die Software kann als eigenständiges einzelnes Applikationsprogramm ausgebildet sein oder als eine multimodulare und optional verteilt auf mehreren Rechnern ausgeführte Software (z.B. Authentifizierungssoftware in Form eines Clouddiensts).

Authentifizierung ist die Verifizierung einer behaupteten Eigenschaft (claim) einer Person durch eine prüfende Instanz (z.B. Authentifizierungssoftware), wobei die Person hierfür Daten an die prüfende Instanz bereitstellt, also eine Authentisierung durchführt. Das Wort Authentifizieren bezeichnet hier den gesamten Vorgang der Bereitstellung der Information (Authentisierung) als auch die Echtheitsprüfung dieser Daten. Die Authentisierung einer Person bezüglich der behaupteten Eigenschaft der Authentizität, die beispielsweise Einräumen einer "bestehenden Zugangsberechtigung" oder "Mitgliedschaft in einer Personengruppe, die bestimmte Rechte zum Zugriff auf bestimmte Funktionen hat", sein kann, erlaubt der authentifizierten Person weitere Aktionen. Die Person gilt dann als authentisch bzw. als "erfolgreich authentifiziert".

### Kurze Beschreibung der Zeichnung

Nachfolgend werden Ausführungsformen der Erfindung mit Bezug auf die Zeichnung beschrieben.

### In der Zeichnung zeigt:

- Fig. 1: ein exemplarisches Flussdiagramm einer Ausgestaltung des erfindungsgemäßen Verfahrens zur Authentifizierung einer Person;
- Fig. 2: ein Blockdiagramm eines Authentifizierungssystems;
- Fig. 3: ein Authentifizierungssystem auf Basis von Bildern in Ausweisdokumenten;
- Fig. 4: ein Authentifizierungssystem auf Basis von Spiegelbildern;
- Fig. 5: ein Authentifizierungssystem auf Basis von Kamerabildern;
- Fig. 6: eine Sequenz von angezeigten Selbstbildern und Fremdbildern;
- Fig. 7: eine Sequenz von ersten und zweiten Hirnaktivitätssignalen;
- Fig. 8: ein Schema eines neuronalen Netzes zur Analyse und Klassifizierung der Hirnaktivitätssignale;
- Fig. 9: einen Plot mit zwei Hirnaktivitätssignalen in Form gemessener Ladungsumverteilungen in der Sensorumgebung bei Betrachtung eines Fremdbilds und eines Selbstbilds;
- Fig. 10: ein Blockdiagramm eines weiteren Authentifizierungssystems; und
- Fig. 11: einen Plot mit Hirnaktivitätssignalen in Form von gemessenen Pupillendurchmesser bei Betrachtung eines Fremdbilds und eines Selbstbilds.

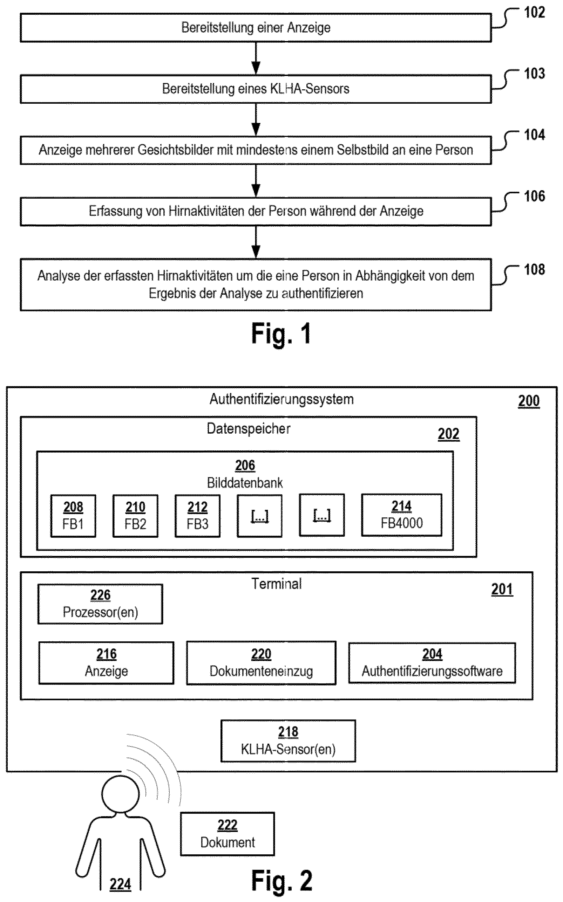

**Figur** 1 illustriert ein exemplarisches Flussdiagramm einer Ausgestaltung des erfindungsgemäßen Verfahrens zur Authentifizierung einer Person.

In einem ersten Schritt 102 wird eine Anzeige bereitgestellt. Beispielsweise kann es sich bei der Anzeige um einen Bildschirm eines Terminals, das zur Prüfung der Identität von Passagieren an einem Flughafen verwendet wird, handeln.

Außerdem wird in Schritt 103 ein KLHA-Sensor bereitgestellt. Der KLHA-Sensor kann auch mehrere Sensoren umfassen. Sofern in dieser Anmeldung von einem einzigen Sensor gesprochen wird, ist die Möglichkeit der Verwendung mehrerer Sensoren implizit mitgemeint. Der Sensor wird so bereitgestellt, dass die Position, Entfernung und Orientierung des Sensors relativ zu dem Kopf der Person, die sich authentifizieren möchte, geeignet ist, dass im jeweiligen Fall zu erfassende Hirnaktivitätssignal in hinreichender Stärke zu erfassen.

Falls es sich bei dem KLHA-Sensor beispielsweise um einen elektrischen Sensor handelt, kann dieser innerhalb der Kopfstütze einer Sitzgelegenheit, auf welche sich die zu authentifizierende Person setzen soll, verbaut sein. Der Sensor ist in diesem Fall in der Nähe des Hinterkopfes der Person in einem Abstand von typischerweise weniger als 15 cm, vorzugsweise weniger als 10 cm, vom Kopf der Person entfernt, ohne allerdings den Kopf direkt zu berühren.

Falls es sich bei dem Sensor um einen optischen Sensor handelt, zum Beispiel eine Kamera, so ist diese beispielsweise so an der Decke oder Wand eines Raumes verbaut, dass die Kamera ein Bild des Gesichtes oder zumindest der Augenpartie der Person erfassen kann, wenn die Person sich an einer bestimmten Position innerhalb des Raumes befindet, an welcher die Authentifizierung stattfinden soll. Beispielsweise kann diese Position durch Markierungen auf dem Fußboden angedeutet sein, wie dies beispielsweise schon heute bei Körperscannern für die Sicherheitskontrolle in Flughafen üblich ist.

In Schritt 104 wird der einen Person, nachdem diese eine für die Authentifizierung geeignete Position in der Nähe des Sensors eingenommen hat, eine Sequenz mehrerer Gesichtsbilder über die Anzeige angezeigt. Die Sequenz umfasst mindestens ein Selbstbild der einen Person, die sich authentifizieren will, und mehrere Fremdbilder. Vorzugsweise umfasst die Sequenz jedoch auch mehrere Selbstbilder, wobei die Sequenz der Selbstbilder und Fremdbilder bei jedem Authentifizierungsvorgang von der Authentifizierungssoftware auf Zufallsbasis neu erzeugt wird.

Eine zufällige Sequenz von Selbst- und Fremdbildern ist eine Bildsequenz, bei welcher die chronologische Reihenfolge der Selbstbilder und Fremdbilder und optional zudem auch die Anzahl der Selbstbilder und/oder Fremdbilder zufällig gewählt wird.

Die zufällig erzeugte Sequenz der Selbstbilder und Fremdbilder fungiert also als Challenge. Im Zuge der Authentifizierung prüft die Authentifizierungssoftware, ob die chronologische Reihenfolge der ersten und zweiten Hirnaktivitätssignale dem in der Challenge codierten zeitlichen Muster von Selbstbildern und Fremdbildern entspricht.

Gemäß Ausführungsformen prüft die Authentifizierungssoftware also, ob zu den Zeiten, in welchen Selbstbilder gezeigt wurden, immer das gleiche oder ein sehr ähnliches erstes Hirnaktivitätssignal empfangen wurde, und ob zu den Zeiten, in welchen Fremdbilder gezeigt wurden, immer das gleiche oder ein sehr ähnliches zweites Hirnaktivitätssignal empfangen wurde, wobei das erste und zweite Hirnaktivitätssignal signifikant unterschiedlich voneinander sind. Nur falls alle diese Kriterien erfüllt sind, wird die Person als erfolgreich authentifiziert behandelt.

Beispielsweise kann dies so erfolgen, dass zunächst mit einem einfachen und nicht notwendigerweise völlig fälschungssicheren Verfahren der Name oder ein sonstiger Identifikator der Person, die sich authentifizieren möchte, festgestellt wird. Beispielsweise kann die Person dazu aufgefordert werden, den eigenen Namen einzugeben, oder es findet eine automatische, konventionelle softwarebasierte Gesichtserkennung statt, oder es wird anhand sonstiger biometrischer Merkmale versucht, den Namen oder Identifikator einer bereits bei der Authentifizierungssoftware registrierten Person zu identifizieren. In einer Datenbank, die der Authentifizierungssoftware zugänglich ist, sind ein oder mehrere Selbstbilder dieser einen registrierten Person hinterlegt. Außerdem beinhaltet die Datenbank mehrere weitere Bilder, die das Gesicht anderer Personen zeigen, beispielsweise die Gesichter anderer registrierter Personen. Diese weiteren Bilder sind Fremdbilder im Hinblick auf die eine Person, die sich authentifizieren möchte. Die Authentifizierungssoftware hat Zugriff auf diese Datenbank und ist dazu konfiguriert, ein oder mehrere Selbstbilder und mehrere Fremdbilder gemäß des Ergebnisses des Zufallsgenerators zufällig auszuwählen und in einer für jeden Authentifizierungsprozess erneut zufällig gewählten zeitlichen Reihung anzuzeigen.

In Schritt 106, welcher zeitgleich bzw. parallel zur Schritt 104 ausgeführt wird, erfasst der KLHA-Sensor die Hirnaktivitäten der Person während die verschiedenen Gesichtsbilder über die Anzeige angezeigt werden. Der Authentifizierungssoftware ist bekannt, zu welchen Zeiträumen Selbstbilder bzw. Fremdbilder angezeigt wurden. Die Authentifizierungssoftware erfasst diejenigen Hirnaktivitätssignale, die jeweils während der Anzeige eines Selbstbildes von dem KLHA-Sensor erfasst wurden, als sogenannte "erste Hirnaktivitätssignale". Hirnaktivitätssignale, die jeweils während der Anzeige eines Fremdbildes von dem KLHA-Sensor erfasst wurden, werden von der Authentifizierungssoftware als sogenannte "zweite Hirnaktivitätssignale" erfasst.

In Schritt 108 analysiert die Authentifizierungssoftware die erfassten ersten und zweiten Hirnaktivitätssignale um festzustellen, ob die ersten und zweiten Hirnaktivitätssignale signifikant unterschiedlich sind, und untersucht außerdem, ob die Signale der gleichen Bildkategorie (Fremdbild bzw. Selbstbild) identisch oder sehr ähnlich zueinander sind. Nur im Fall dass die ersten und zweiten Hirnaktivitätssignale signifikant unterschiedlich sind, und wenn Hirnaktivitätssignale der gleichen Bildkategorie identisch oder sehr ähnlich sind, behandelt die Authentifizierungssoftware die eine Person als erfolgreich authentifiziert. Beispielsweise kann dies bewirken, dass die Authentifizierungssoftware der einen Person nach erfolgreiche Authentifikation Zugriff zu sensiblen Daten, Zugriff auf sensible Software- oder Hardwarefunktionen und/oder Zutritt zu geschützten Räumlichkeiten, Gebäuden oder geographischen Bereichen gewährt.

Ausführungsformen der Erfindung können in vielfältigen Bereichen zum Einsatz kommen, zum Beispiel für Identitätskontrollen an Flughäfen, Firmentoren, und/oder Ländergrenzen. Da kein Kontakt zwischen der zu authentifizierenden Person und dem Sensor erforderlich ist, ist das Verfahren besonders geeignet zur schnellen Identitätsprüfung einer großen Zahl von Personen in kurzer Zeit.

**Figur** 2 zeigt ein Blockdiagramm eines Authentifizierungssystems 200.

Das Authentifizierungssystem beinhaltet eine Anzeige 216, zum Beispiel einen elektronischen Monitor, der beispielsweise Bestandteil eines Terminals 201 sein kann. Das Terminal beinhaltet ein oder mehrere Prozessoren 226, die dazu ausgebildet sind, computerinterpretierbare Instruktionen einer Authentifizierungssoftware 204, die auf einem Speicher des Terminals gespeichert ist, auszuführen. Das Terminal kann über einen Dokumenteneinzug 220 verfügen, über welchen ein Identitätsnachweis 222 einer Person 224, die sich authentifizieren möchte, zum Beispiel ein Personalausweis oder Reisepass, eingezogen werden kann.

Das Authentifizierungssystem 200 umfasst zudem einen KLHA Sensor 218 und einen Datenspeicher 202, in welchem sich eine Bilddatenbank 206 befindet. Der Datenspeicher kann Bestandteil des Terminals 201 sein, oder mit dem Terminal über ein Netzwerk, zum Beispiel ein Intranet oder das Internet, verbunden sein. Die Bilddatenbank kann eine Vielzahl von Gesichtsbildern von Personen enthalten. Beispielsweise kann es sich um Gesichtsbilder von Personen handeln, die beim Betreiber des Terminals "registriert" oder auf sonstige Art und Weise bekannt oder verzeichnet sind. Bei den Personen kann es sich zum Beispiel um die Mitarbeiter eine Firma oder die Bürger eines Landes handeln. Die Bilddatenbank beinhaltet beispielsweise ein Fremdbild 208, ein weiteres Fremdbild 210, sowie eine Vielzahl weiterer Fremdbilder 212, 214. In manchen Ausführungsformen, insbesondere in denen, in welchen die Selbstbilder nicht durch eine Spiegelvorrichtung oder eine Kamera erzeugt bzw. erfasst werden, beinhaltet die Bilddatenbank auch ein oder mehrere Selbstbilder (hier nicht gezeigt). Ob es sich bei einem Bild in der Bilddatenbank um ein Selbstbild oder Fremdbild handelt, ist keine statische Eigenschaft des jeweiligen Bildes, sondern hängt von der Person ab, die sich zu einem bestimmten Zeitpunkt authentifizieren möchte. Wenn ein Bild der Datenbank das Gesicht dieser Person abbildet, handelt es sich um ein Selbstbild, andernfalls um ein Fremdbild.

Die Authentifizierungssoftware 204 ist dazu ausgebildet, für jeden Authentifizierungsvorgang eine neue zeitliche Sequenz aus Selbstbildern und Fremdbildern zu erzeugen und gemäß dieser Sequenz über die Anzeige 216 auszugeben. Die Authentifizierungssoftware ist außerdem dazu ausgebildet, die während der Anzeige der verschiedenen Bilder vom KLHA-Sensor 218 erfassten Hirnaktivitätssignale zu analysieren um festzustellen, ob die ein oder mehreren ersten Hirnaktivitätssignale, die während der Anzeige von Selbstbildern empfangen wurden, signifikant unähnlich sind zu mehreren zweiten Hirnaktivitätssignalen, die während der Anzeige von Fremdbildern empfangen wurden , wobei Hirnaktivitätssignale, die während der Anzeige von Gesichtsbildern des gleichen Typs erfasst werden, vorzugsweise identisch oder sehr ähnlich sein müssen. Je nach Art der erfassten Hirnaktivitätssignale kann diese Analyse recht unterschiedlich ausfallen. Beispielsweise kann die Analyse eine Bestimmung und einen Vergleich von Amplitude und/oder Amplitudenverlauf über die Zeit ("Signalprofil") eines gemessenen ersten und zweiten Signals umfassen. Bei dem Signal kann es sich um ein elektrisches Signal (gemessen z.B. in Volt) oder ein optisch und durch Bildanalyse erfasstes Signal (gemessen z.B. in mm Pupillendurchmesser bzw. Pupillendurchmesseränderung) handeln. Die Person 224 gilt als erfolgreich gegenüber der Authentifizierungssoftware authentifiziert, wenn die Authentifizierungssoftware feststellt, dass die ersten Hirnaktivitätssignale signifikant unterschiedlich sind zu den zweiten Hirnaktivitätssignalen, wobei die Hirnaktivitätssignale, die beim Anzeigen von Bildern des gleichen Typs empfangen wurden, vorzugsweise identisch oder sehr ähnlich sind.

Es ist also nicht in jedem Fall erforderlich, dass zum Beispiel die ersten Hirnaktivitätssignale bei allen Menschen identisch oder nahezu identisch sind. Die Anmelderin hat jedoch festgestellt, dass zumindest eine deutliche Unähnlichkeit der Hirnaktivitätssignale bei Selbstbildern und Fremdbildern bei praktisch allen Menschen in reproduzierbarer Weise gegeben ist. Somit kann gemäß Ausführungsformen der Erfindung ein Authentifizierungsverfahren bereitgestellt werden, welches die Vorzüge biometrischer Authentifizierungsverfahren mit den Vorzügen von Challenge Response-Authentifizierungsverfahren verbindet.

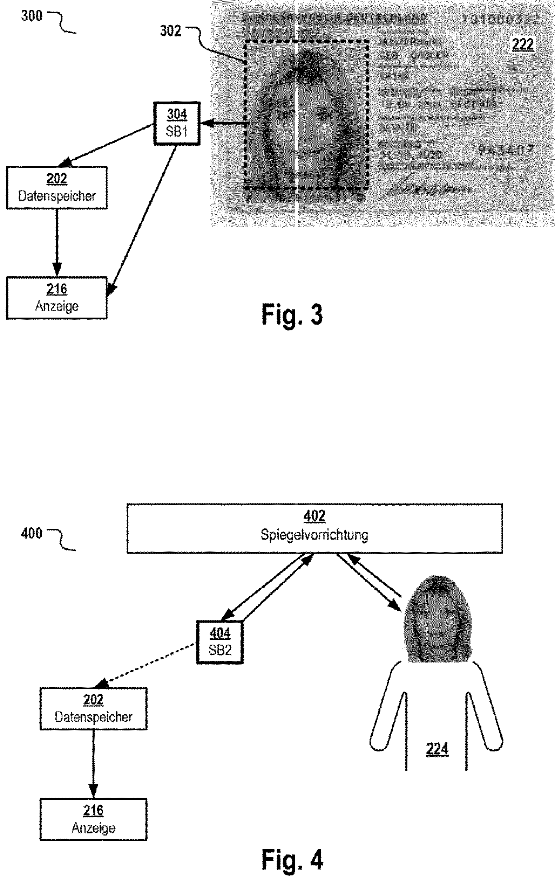

**Figur** 3 zeigt ein Authentifizierungssystem 300 auf Basis von Bildern in Ausweisdokumenten. Beispielsweise kann es sich bei dem Ausweisdokument 222 um einen Personalausweis handeln, der über einen Dokumenteneinzug 220 in ein Terminal eingezogen und dort ausgewertet wird. Das Terminal kann zum Beispiel eine weitere Kamera beinhalten, die das auf dem Dokument 222 abgebildete Gesichtsbild 302 aufnimmt und dieses Bild als Selbstbild 304 der zu authentifizierenden Person verwendet. Beispielsweise kann das Bild zunächst in einem Datenspeicher 202, zum Beispiel innerhalb der Bilddatenbank 206, gespeichert werden und dann im nächsten Schritt als Bestandteil einer zufällig gewählten Sequenz mehrerer Selbstbilder und Fremdbilder über die Anzeige 216 angezeigt zu werden. Beispielsweise kann das Selbstbild 304 ein oder mehrmals dupliziert werden, sodass mehrere Kopien des Selbstbilds 304 vorhanden sind und eine komplexe Sequenz aus Selbstbildern und Fremdbildern erzeugt werden kann. Das Authentifizierungssystem 300 verwendet also sowohl eine Bilddatenbank als auch das in einem Ausweisdokument der sich authentifizierenden Person abgedruckten Gesichtsbild, oben für den aktuellen Authentifizierungsprozess eine zufällige Sequenz aus Fremdbildern und Selbstbildern zu erzeugen, die der Person über die Anzeige angezeigt werden.

**Figur 4** zeigt ein Authentifizierungssystem 400 auf Basis von Spiegelbildern. Das System 400 beinhaltet eine Vorrichtung 402 mit einem oder mehreren Spiegeln, die so angeordnet sind, dass die Person, die sich authentifiziert, ihr eigenes Spiegelbild zumindest zeitweise erblickt. Beispielsweise kann die Spiegelvorrichtung 402 einen semi-transparenten Spiegel beinhalten, der dann, wenn das Gesicht der Person 224 von einer bestimmten Lichtquelle beleuchtet wird, dieser Person 224 ihr eigenes Gesichts-Spiegelbild zeigt. Hinter dem semi-transparenten Spiegel ist eine elektronische Digitalanzeige angebracht, die zumindest dann, wenn die Lichtquelle angeschaltet/aktiv ist, ausgeschaltet ist oder nur ein so schwaches Lichtsignal emittiert, dass die Person 224 kein Bild auf diese Digitalanzeige wahrnehmen kann. Die Authentifizierungssoftware ist dazu konfiguriert, die Aktivität der Lichtquelle und die Helligkeit der digitalen Anzeige hinter dem semi-transparenten Spiegels so zu koordinieren, dass dann, wenn auf der Digitalanzeige Fremdbilder angezeigt werden, die Lichtquelle, die das Gesicht der Person 224 bestrahlt, deaktiviert wird und gleichzeitig die digitale Anzeige mit einem angezeigten Fremdbild aktiviert wird. Das Aktivieren der Lichtquelle bei deaktivierter digitaler Anzeige und das Aktivieren der digitalen Anzeige bei deaktivierter Lichtquelle wird von der Authentifizierungssoftware gemäß der für jeden Authentifizierungsvorgang neu erzeugten zufällige Sequenz aus Selbstbildern und Fremdbildern so orchestriert, dass die Lichtquelle immer dann aktiviert und die digitale Anzeige immer dann deaktiviert wird, wenn der Person ein Selbstbild (nämlich ihr Spiegelbild) gezeigt werden soll, und dass die Lichtquelle immer dann deaktiviert und die digitale Anzeige immer dann aktiviert wird, wenn der Person über die digitale Anzeige ein Fremdbild angezeigt werden soll.

Das über die Spiegelvorrichtung 402 erzeugte Selbstbild 404 wird typischerweise direkt über die Spiegelvorrichtung, die als Anzeige 216 für die Selbstbilder wirkt, angezeigt. Optional kann das Selbstbild 404 zusätzlich von einer Kamera erfasst und in dem Datenspeicher 202, der die Fremdbilder für die Digitalanzeige 216 umfasst, gespeichert werden.

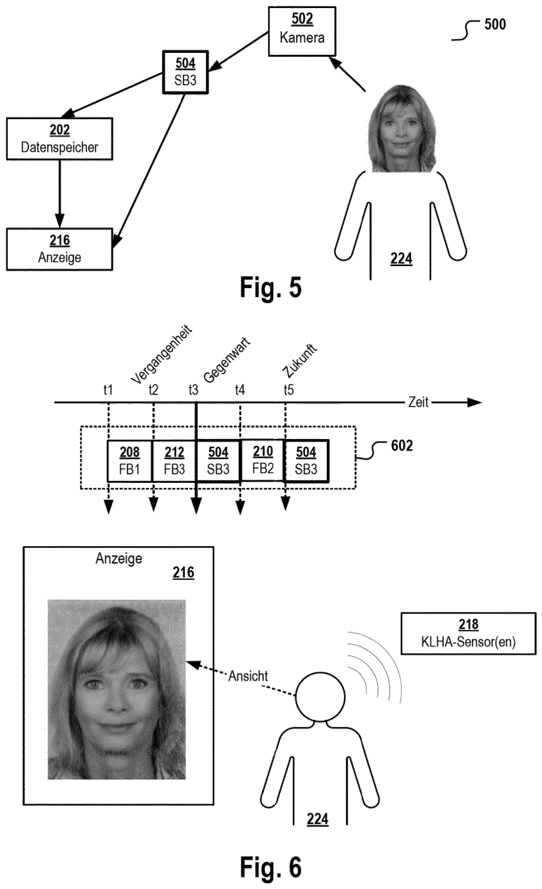

**Figur 5** zeigt ein Authentifizierungssystem 500 auf Basis von Kamerabildern. Eine Kamera 502 erfasst das Gesichtsbild 504 der einen Person 224, die sich authentifizieren möchte, und zeigt es der Person 224 über die digitale Anzeige 216 an.

Die Authentifizierungssoftware ist dazu konfiguriert, für jeden Authentifizierungsprozess eine neue, zufällige Sequenz aus mehreren Selbstbildern und Fremdbildern zu erzeugen. Beispielsweise kann die Authentifizierungssoftware nach der Erfassung des Selbstbildes 504 mit der Kamera zunächst eine oder mehrere Kopien des Selbstbildes erzeugen. Dann liest die Authentifizierungssoftware mehrere Fremdbilder aus einer Bilddatenbank 206 aus und kombiniert die Kopien des Selbstbildes mit den ausgewählten Fremdbildern zu der neuen Bildsequenz. Die Bilder werden der Person 224 gemäß dieser Sequenz über die Anzeige 216 angezeigt. Währenddessen erfasst der KLHA-Sensor die Hirnaktivitätssignale der Person und übermittelt sie der Authentifizierungssoftware zur weiteren Analyse und Authentifizierung der Person.

Optional kann das erfasste Selbstbild 504 auch in dem Datenspeicher 202 gespeichert werden, beispielsweise, damit das Selbstbild 504 als Fremdbild im Zuge der Authentifikation anderer Personen verwendet werden kann.

**Figur 6** zeigt eine Sequenz 602 von angezeigten Selbstbildern und Fremdbildern, die von der Authentifizierungssoftware für einen aktuellen Authentifizierungsprozess der Person 224 zufällig erzeugt wurde. Die Sequenz beginnt mit einem ersten Fremdbild 208. Es folgt ein weiteres Fremdbild 212, dann ein Selbstbild 504, dann ein anderes Fremdbild 210 und schließlich wieder ein Selbstbild 504.

Die Authentifizierungssoftware ist dazu ausgebildet, gemäß dieser zufällig erzeugten Sequenz die einzelnen Bilder nacheinander über die Anzeige 216 der Person 224 anzuzeigen. Beispielsweise kann die Authentifizierungssoftware dazu konfiguriert sein, jedes der Bilder 208, 212, 504, 210, 504 zwei Sekunden lang anzuzeigen und nach Ablauf der zwei Sekunden ohne Pause sofort das nächste Bild der Sequenz anzuzeigen. Beispielsweise zeigte die Authentifizierungssoftware an dem in der Vergangenheit liegenden Zeitpunkt t1 das Fremdbild 208 an, 2 Sekunden später, zum Zeitpunkt t2, ein weiteres Fremdbild einer anderen fremden Person, und wiederum 2 Sekunden später, zum Zeitpunkt t3, ein Selbstbild 504 der Person 224, die sich aktuell authentifizieren möchte. Die Anzeige 216 zeigt das zum aktuellen Zeitpunkt (zwischen t3 und t4) auf der Anzeige ausgegebene Selbstbild der Person. Wenn nach dem Zeitpunkt t3 weitere 2 Sekunden verstrichen sein werden, wird das Selbstbild 504 durch das weitere Fremdbild 210 ersetzt werden, und nach weiteren 2 Sekunden zum Zeitpunkt t5 durch das Selbstbild 504. Während die Bildsequenz 602 der Person 224 angezeigt wird, wird eine entsprechende Sequenz von ersten und zweiten Hirnaktivitätssignalen vom KLHA-Sensor aufgezeichnet und an die Authentifizierungssoftware zur weiteren Analyse und zur Authentifizierung der Person weitergeleitet.

Die Anzeigedauer der jeweiligen Bilder liegt typischerweise im Bereich zwischen einer und 5 Sekunden, vorzugsweise im Bereich 2-4 Sekunden. Es ist möglich, dass Pausen zwischen dem Anzeigen verschiedener Bilder eingeführt werden, die typischerweise kürzer sind als 5 Sekunden.

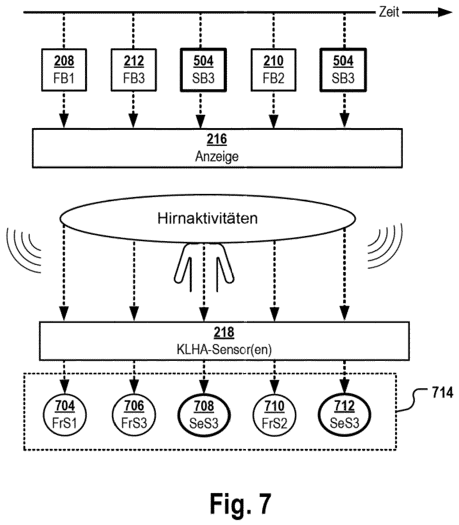

**Figur** 7 zeigt eine Sequenz 714 von ersten und zweiten Hirnaktivitätssignalen, die durch Anzeige der Bildsequenz 602 in der Person 224 hervorgerufen werden.

Die Anzeige des Fremdbilds 208 erzeugt ein zweites Hirnaktivitätssignal 704. Die Anzeige des Fremdbilds 212 erzeugt ein zweites Hirnaktivitätssignal 706. Die Anzeige des auf das Bild 212 folgenden Selbstbildes 504 erzeugt ein erstes Hirnaktivitätssignal 708. Die Anzeige des Fremdbilds 210 erzeugt ein zweites Hirnaktivitätssignal 710. Die Anzeige des auf das Bild 210 folgenden Selbstbildes 504 erzeugt ein erstes Hirnaktivitätssignal 712. Die gestrichelten Pfeile repräsentieren hier nicht Zeitpunkte, sondern Zeitfenster, zum Beispiel 2 Sekunden lange Zeitfenster, während welcher die entsprechenden Bilder angezeigt und die zu diesen korrespondierenden Hirnaktivitätssignale gemessen werden.

Wenn die Person 224 keine Maske trägt bzw. nicht versucht, sich mit einem Ausweis mit einem gemorphten Bild zu authentifizieren, so ist zu erwarten, dass alle ersten Hirnaktivitätssignale 708, 712 einander sehr ähnlich oder identisch sind, dass alle zweiten Hirnaktivitätssignale 704, 706, 710 einander sehr ähnlich oder identisch sind, und dass die ersten und zweiten Hirnaktivitätssignale signifikant unterschiedlich sind. In diesem Fall behandelt die Authentifizierungssoftware die Person 224 als erfolgreich authentifiziert. Andernfalls behandelt die Authentifizierungssoftware die Person als nicht authentifiziert und verweigert den begehrten Zugang bzw. die begehrte Aktion.

Ein praktisches Anwendungsszenario gemäß einer Ausführungsform der Erfindung wird im Folgenden beschrieben: Beispielsweise wird das Authentifizierungsverfahren zur Authentifizierung eines Mitarbeiters beim Betreten des Firmengeländes verwendet. In der Firma arbeiten 500 Mitarbeiter M1-M500. In einer Datenbank sind für jeden Mitarbeiter zehn Gesichtsbilder des jeweiligen Mitarbeiters hinterlegt, also für den Mitarbeiter M1 die Gesichtsbilder B_{M1.1}, B_{M1.2}, ..., BM_{1.10}, für Mitarbeiter M2 die Gesichtsbilder BM_{2.1}-BM_{2.10}. Der erste Mitarbeiter M1 möchte sich authentifizieren und stößt ein erstes Authentifizierungsverfahren an. Im Zuge dessen wählt die Authentifizierungssoftware aus der Bilddatenbank eine zufällige Sequenz aus zwei Selbstbilder und vier Fremdbildern aus, z.B. gemäß der Sequenz B_{M44.7}, B_{M102.2}, B_{M1.3}, B_{M15.1}, B_{M1.8}, B_{M16.7}. In dieser Sequenz sind die Bilder B_{M1.3} und B_{M1.8} "Selbstbilder" bezüglich Mitarbeiter M1. Die Bilder der anderen Mitarbeiter M44, M102, M15 und M16 sind Fremdbilder für Mitarbeiter M1, können jeweils aber als Selbstbilder dienen, wenn sich diese anderen Mitarbeiter authentifizieren. Sofern es sich bei dem sich gerade authentifizierenden Mitarbeiter tatsächlich um Mitarbeiter M1 handelt, ist also folgende Signalsequenz zu erwarten, wobei die Kürzel "HAS1" für "erstes Hirnaktivitätssignal" und HAS2" für "zweites Hirnaktivitätssignal" verwendet werden: HAS2, HAS2, HAS1, HAS2, HAS1, HAS2. Wenn die Abfolge der erfassten ersten und zweiten Hirnaktivitätssignale den gemäß der Sequenz der gezeigten Gesichtsbilder erwarteten Signalabfolge entspricht, hat sich der Mitarbeiter M1 erfolgreich authentifiziert.

Der erste Mitarbeiter M1 möchte sich authentifizieren und stößt ein erstes Authentifizierungsverfahren an. Im Zuge dessen wählt die Authentifizierungssoftware aus der Bilddatenbank eine zufällige Sequenz aus zwei Selbstbildern und vier Fremdbildern aus, z.B. gemäß der Sequenz B_{M44.7}, B_{M102.2}, B_{M1.3}, B_{M15.1}, B_{M18}, B_{M16.7}. In dieser Sequenz sind die Bilder B_{M1.3} und B_{M1.8} "Selbstbilder" bezüglich Mitarbeiter M1. Die Bilder der anderen Mitarbeiter M44, M102, M15 und M16 sind Fremdbilder für Mitarbeiter M1, können jeweils aber als Selbstbilder dienen, wenn sich diese anderen Mitarbeiter authentifizieren. Sofern es sich bei dem sich gerade authentifizierenden Mitarbeiter tatsächlich um Mitarbeiter M1 handelt, ist also folgende Signalsequenz zu erwarten, wobei die Kürzel "HAS1" für "erstes Hirnaktivitätssignal" und HAS2" für "zweites Hirnaktivitätssignal" verwendet werden: HAS2, HAS2, HAS1, HAS2, HAS1, HAS2.

Danach möchte sich Mitarbeiter M55 authentifizieren und stößt ein zweites Authentifizierungsverfahren an. Im Zuge dessen wählt die Authentifizierungssoftware aus der Bilddatenbank eine zufällige Sequenz aus zwei Selbstbildern und vier Fremdbildern aus, z.B. gemäß der Sequenz B_{M55.2}, B_{M14.7}, B_{M55.6}, B_{M232.2}, B_{M15.1}, B_{M46.7}. In dieser Sequenz sind die Bilder B_{M55.2} und B_{M55.6} "Selbstbilder" bezüglich Mitarbeiter M55. Die Bilder der anderen Mitarbeiter M14, M232, M15 und M46 sind Fremdbilder für Mitarbeiter M55, können jeweils aber als Selbstbilder dienen, wenn sich diese anderen Mitarbeiter authentifizieren. Sofern es sich bei dem sich gerade authentifizierenden Mitarbeiter tatsächlich um Mitarbeiter M55 handelt, ist also folgende Signalsequenz zu erwarten: HAS1, HAS2, HAS1, HAS2, HAS2, HAS2.

**Figur 8** zeigt ein Schema einer Machine-Learning-Software zur Analyse und Klassifizierung der Hirnaktivitätssignale, um festzustellen, ob und wie sehr sich erste und zweite Hirnaktivitätssignale gegenseitig und untereinander ähneln. Es hat sich herausgestellt, dass neuronale Netze , insbesondere rekurrente neuronale Netze 804, besonders geeignet sind, um die Ähnlichkeit von Hirnaktivitätssignalen zu bestimmen.

Beispielsweise kann die Machine-Learning-Software neben dem neuronalen Netz 804 ein Vorverarbeitung Modul 802 und ein Auswertungsmodul 806 besitzen. Das Vorverarbeitung Modul 802 ist dazu konfiguriert, das von dem KLHA-Sensor 218 erfasste Signal so zu repräsentieren, dass die in dem Signal enthaltene Information von dem neuronalen Netz ausgewertet werden kann. Beispielsweise kann das Profil zeitlicher Änderungen von Amplitude (gemessene Ladungsänderungen und/oder von Ladungsumverteilungen in der Nähe eines elektrischen Sensors) und optional auch Frequenz des von einem elektrischen Sensor gemessenen Signals als mehrdimensionaler Vektor dargestellt werden, wobei beispielsweise der Vektor eine Vielzahl von Elementen (z.B. 200 Elemente für einen Vektor, der Signaleigenschaften eines über zwei Sekunden gemessenen Signals mit einer zeitlichen Auflösung von 10 ms spezifiziert) umfassen kann, wobei pro Element ein zu diesem Zeitpunkt gültige Amplitudenwert und eine zu diesem Zeitpunkt gemessene Frequenz in den Vektor enthalten ist. Falls es sich bei dem KLHA-Sensor um einen optischen Sensor handelt, kann das Vorverarbeitungsmodul 802 dazu ausgebildet sein, aus dem von dem optischen Sensor erfassten Gesichtsbild bzw. Augenbereichsbild mehrere Merkmale zu extrahieren wie zum Beispiel Pupillendurchmesser und/oder zeitliches Profil von Pupillendurchmesseränderungen, und diese Merkmale in geeigneter Form zu repräsentieren, zum Beispiel ebenfalls als Vektor mit mehreren numerischen Werten definierte Bedeutung. In einer Trainingsphase hat die Machine-Learning-Software 800 auf Basis eines annotierten Trainingsdatensatzes gelernt, zu erkennen, welche Variabilität der Signaleigenschaften akzeptiert werden kann und muss, ohne dass zwei verglichene Hirnaktivitätssignale als signifikant unähnlich angesehen werden. Biometrische Signale sind niemals 100 % identisch. Es ist aber möglich, durch das Training einer Machine-Learning Software auf einen Datensatz mehrerer Hirnaktivitätssignale, die einmal durch Betrachtung von Selbstbildern und ein anderes Mal durch Betrachtung von Fremdbildern hervorgerufen wurden, und die entsprechend annotiert sind, zu erreichen, dass die trainierte Machine-Learning Software zuverlässig erkennen kann, ob Abweichungen in Eigenschaften der Hirnaktivitätssignale relevant oder irrelevant im Hinblick auf die Frage sind, ob hier ein Selbstbild oder ein Fremdbild betrachtet wird.

Das Auswertungsmodul 806 aggregierten die Ausgaben der Neuronen der Ausgabeschicht des neuronalen Netzes und bereitet das Ergebnis (authentifiziert oder nicht) so auf, dass dies vom Empfänger interpretiert werden kann. Beispielsweise kann das Ergebnis als natürlichsprachlicher Text akustisch oder optisch an einen Menschen ausgegeben werden. Alternativ oder zusätzlich kann das Ergebnis der Authentifizierung auch an eine Software oder Hardwarekomponente ausgegeben werden, die in Abhängigkeit von dem Authentifizierungs-Ergebnis beispielsweise eine bestimmte Hardwarefunktion oder Softwarefunktion freigibt oder nicht.

Gemäß einer anderen, hier nicht dargestellten Ausführungsform dient ein Convolutional Neural Network (CNN) zur Klassifikation der Hirnaktivitätssignale in ähnliche und unähnliche Signale. Es kann z.B. eine erste Faltungsschicht mit sechs Feature-Maps, deren Kernelgröße 5 ist, beinhalten. Die erste Faltungsschicht ist eine erste Maxpooling-Schicht mit der Skalengröße 2. Eine zweite Faltungsschicht mit 12 Merkmalspunkten hat die Kernelgröße 3. Außerdem beinhaltet das neuronale Netz eine zweite Maxpooling-Schicht mit der gleichen Skalengröße wie die erste Maxpooling-Schicht. Schließlich gibt es eine voll verbundene Schicht mit 108 Merkmalspunkten, die die Score-Schätzung der Eingabe berechnet. Im Trainingsstadium werden Lernrate, Lossgröße und Lernepoche z.B. auf 1, 100 und 500 gesetzt.

**Figur 9** zeigt einen Plot 900 mit einem ersten Hirnaktivitätssignal 902, das während der Betrachtung eines Selbstbildes von einem elektrischen Sensor gemessen wurde, und mit einem zweiten Hirnaktivitätssignal 904, dass während der Betrachtung eines Fremdbildes von einem elektrischen Sensor gemessen wurde. Bei den hier gezeigten Hirnaktivitätssignalen handelt es sich um Hirnstromsignale, die kontaktlos in Form von Ladungsänderungen und/oder von Ladungsumverteilungen in der Nähe des elektrischen Sensors erfasst wurden.

Generell unterscheidet man in der Neurologie bei Hirnstrommessungen unterschiedliche Frequenzbänder:
- Das Sub-δ-Band mit einem Frequenzbereich von 0.15 bis 0.5 Hz,
- das δ-Band mit einem Frequenzbereich von 0.5 bis 3.5 Hz,
- das θ -(Theta)-Band mit einem Frequenzbereich von 3.5 bis 8 Hz,
- das α-Band mit einem Frequenzbereich von 8 bis 13 Hz,
- das β-Band mit einem Frequenzbereich von 13 bis 30 Hz, sowie Frequenzanteile oberhalb des β-Bandes (also über 30 Hz).

Gemäß Ausführungsformen werden die Hirnaktivitätssignale in einem oder mehreren dieser Bänder gemessen.

Das α-Band liefert in manchen Ausführungsformen zusätzliche Informationen über den Wachheitszustand einer Person, Frequenzanteile oberhalb dieses α -Bandes (β-Band und darüber) können Informationen über Beeinträchtigung der mentalen Funktionen durch Drogen oder zentral wirksame Medikamente liefern, und das direkt unterhalb des α-Bands angrenzende Theta-Band liefert Informationen anderer Art, die u.a. mit dem Wachheitszustand, eingenommenen Substanzen oder Krankheiten korrelieren können. Spezifische Stimuli, z.B. Gesichtsbilder, lösen oftmals Hirnstromsignale im theta-Band und weiteren Bändern aus. Gemäß Ausführungsformen der Erfindung wird eine "Multi-Band"-Signalanalyse, d.h. eine Analyse der Eigenschaften von Signalen über einen sehr breiten Frequenzbereich, durchgeführt.

Gemäß weiteren Ausführungsformen werden die Hirnaktivitätssignale ohne Differenzierung nach einzelnen Bändern gemessen. Vielmehr wird die Signalamplitude über ein weites Frequenzband, das im Wesentlichen nur durch die Sensitivität des elektrischen Sensors bestimmt ist, erfasst.

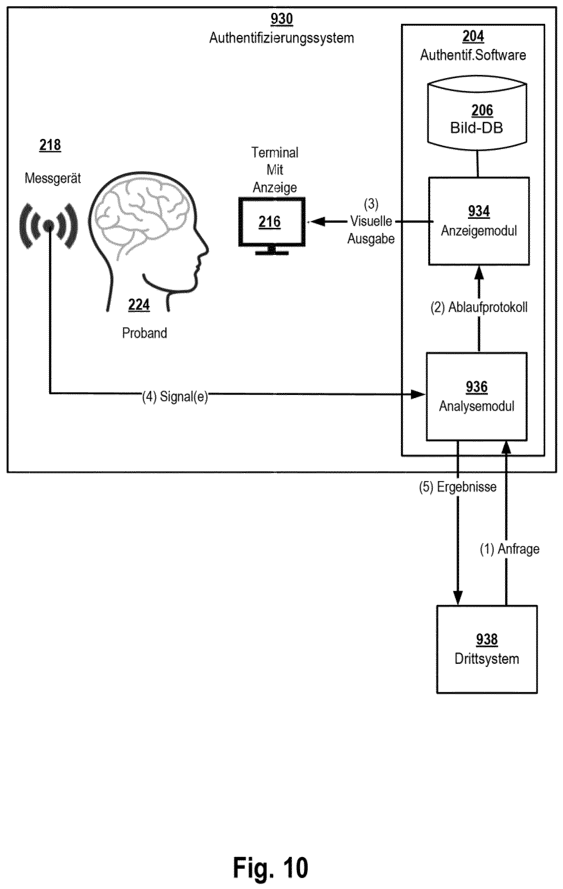

**Figur 10** zeigt ein Blockdiagramm eines weiteren Authentifizierungssystems 930. Der Aufbau des Authentifizierungssystems 930 entspricht im Wesentlichen dem Aufbau des Authentifizierungssystems 200, das in Figur 2 beschrieben ist. Die Person 224 ist nicht Bestandteil des Authentifizierungssystems. Figur zehn zeigt, wie das Authentifizierungssystem von einem Drittsystem 938 verwendet werden kann. Beispielsweise kann es sich bei dem Drittsystem um eine Software zur Kontrolle einer Schließanlage, zum Beispiel eine Tür oder eines Tors, handeln. Der Nutzer 224, hier als Proband bezeichnet, versucht, sich bei dem Drittsystem zu authentifizieren, um die Tür zu öffnen. Beispielsweise gibt der Proband 224 seinen Namen in eine Software des Drittsystems ein oder das Drittsystem liest eine Mitarbeiter-ID aus einem Identitätsdokument, zum Beispiel einem Mitarbeiterausweis, aus. Daraufhin sendet das Drittsystem eine Anfrage an die Authentifizierungssoftware 204. Die Anfrage kann beispielsweise den eingegebenen Namen oder die ausgelesenen Mitarbeiter-ID beinhalten. Die Authentifizierungssoftware erzeugt zunächst mithilfe eines Zufallsgenerators eine neue, zufällige Sequenz mehrerer Selbstbilder und mehrerer Fremdbilder. Hierzu greift sie auf eine Bilderdatenbank 206 zu, in welcher mehrere für den Mitarbeiter jeweils mit ein oder mehreren Gesichtsbildern hinterlegt sind. Anhand des eingegebenen Namens bzw. der Mitarbeiter-ID kann die Authentifizierungssoftware erkennen, welche der Bilder in der Datenbank 206 als Selbstbilder und welche als Fremdbilder verwendet werden können. Nachdem die Authentifizierungssoftware eine entsprechende Bildsequenz definiert hat, steuert ein Anzeigemodul 934 der Authentifizierungssoftware die Anzeige der Bilder auf der Anzeige 216 so, dass die Bilder chronologisch gemäß der zufälligen Sequenz dem Nutzer 224 angezeigt werden. Vorzugsweise wird hierbei durch weitere Module der Software 204 in Interoperation mit einer Kamera sichergestellt, dass der Proband 224 den Blick auch wirklich auf die Anzeige 216 richtet, während die Bildsequenz angezeigt wird. Während die Bildsequenz angezeigt wird, erfasst der KLHA-Sensor 218, hier als Messgerät bezeichnet, die von der Person 224 ausgesendeten Hirnaktivitätssignale. Bei diesen Signalen kann es sich um Hirnstromsignale handeln, die die neuronalen Prozesse im Hirn des Probanden direkt wiedergeben, oder um Eigenschaften der Augen, insbesondere der Pupille und der Augenlider, die indirekt die neuronalen Prozesse während des Betrachtens verschiedener Bilder widerspiegeln. Die vom Sensor erfassten Signale werden an ein Analysemodul 936 der Authentifizierungssoftware 204 weitergegeben, wo diese dann aufbereitet und analysiert werden. Beispielsweise kann es sich bei dem Analysemodul 936 um eine Machine-Learning-Software 800 handeln wie diese mit Referenz auf Figur 8 beschrieben ist. In Abhängigkeit davon, ob die Hirnaktivitätssignale, die beim Betrachten von Fremdbildern empfangen wurden, signifikant unterschiedlich sind von den Hirnaktivitätssignalen, die beim Betrachten von Selbstbildern empfangen wurden, entscheidet das Analysemodul 936, ob der Proband 224 sich erfolgreich gegenüber der Authentifizierungssoftware 204 authentifiziert hat. Das Ergebnis dieser Entscheidung wird an das Drittsystem 938 zurückgegeben. Das Drittsystem führt nun seine Funktion in Abhängigkeit dieses Ergebnisses aus. Beispielsweise öffnet eine Kontrollsoftware einer Schließanlage dem Nutzer 224 die Schließanlage, falls sich der Nutzer erfolgreich authentifiziert hat. Andernfalls unterbleibt die Öffnung.

**Figur 11** zeigt einen Plot 950 mit einem ersten Hirnaktivitätssignal 952 in Form eines Pupillendurchmesserprofils, das während der Betrachtung eines Selbstbildes von einem optischen Sensor gemessen wurde, und mit einem zweiten Hirnaktivitätssignal 904 in Form eines zweiten Pupillendurchmesserprofils, dass während der Betrachtung eines Fremdbildes von dem optischen Sensor gemessen wurde. Die beiden Pupillendurchmesserprofile bzw. die jeweiligen Durchmesser wurden durch automatische Bildanalyse der erfassten Bilder ermittelt. Eine Betrachtung eines Selbstbildes führt im Gegensatz zu der Betrachtung eines Fremdbildes bei der hier betrachteten Person zu einer deutlichen Pupillendurchmesservergrößerung innerhalb der ersten 1000 ms.

Das erfindungsgemäße Authentisierungsverfahren ist nicht auf die beschriebenen Ausführungsbeispiele beschränkt. Es kann mit weiteren Authentisierungsverfahren kombiniert werden, um eine sichere und bequeme Authentisierung zu ermöglichen. Eine Kombination mehrere biometrischer Verfahren ist vorteilhaft um eine multimodale Authentifizierung zu gewährleisten.

Vorteilhafte Ausführungsformen umfassen beispielsweise die folgenden als Klauseln formulierte Merkmale:
1. Verfahren zur Authentifizierung einer Person (224), umfassend:
   - Bereitstellung (102) einer Anzeigevorrichtung (216);
   - Bereitstellung (103) eines Sensors (218) zur Sensierung von Hirnaktivitäten, wobei der Sensor (218) ein kontaktloser Sensor (218) ist, wobei der Sensor (218) nachfolgend als KLHA-Sensor (218) bezeichnet wird;
   - Anzeige (104) mehrerer Gesichtsbilder (208-214,304, 404, 504), die Gesichter mehrerer unterschiedlicher Personen abbilden, auf der Anzeige für die eine Person (224), wobei die mehreren Gesichtsbilder ein oder mehrere Selbstbilder (304, 404, 504) und ein oder mehrere Fremdbilder umfassen, wobei ein Selbstbild ein Gesichtsbild dieser einen Person ist, wobei ein Fremdbild ein Gesichtsbild einer anderen der mehreren unterschiedlichen Personen ist, wobei das Anzeigen der mehreren Gesichtsbilder umfasst:
      - für jeden Authentifizierungsvorgang, Erzeugung einer zufälligen Reihung (602) der mehreren Gesichtsbilder durch die Authentifizierungssoftware und Durchführen des Anzeigens der mehreren Gesichtsbilder in einer chronologischen Sequenz entsprechend der zufälligen Reihung; und/oder
      - für jeden Authentifizierungsvorgang, zufällige Auswahl zumindest der ein oder mehreren Fremdbilder aus einer Gesichtsbilddatenbank (206) durch die Authentifizierungssoftware und Durchführen des Anzeigens der mehreren Gesichtsbilder einschließlich der ausgewählten Fremdbilder; wobei die für jeden Authentifizierungsvorgang erzeugte zufällige Reihung als Challenge dient;
   - während die mehreren Gesichtsbilder der einen Person angezeigt werden, Erfassung (106) von ersten (708, 712, 902) und zweiten (704, 706, 710, 904) Hirnaktivitätssignalen, die in der einen Person durch die Betrachtung der Gesichtsbilder jeweils ausgelöst werden, durch den KLHA-Sensor, wobei die ersten Hirnaktivitätssignale erfasst werden, während dieser einen Person die ein oder mehreren Selbstbilder (304, 404, 504) angezeigt werden, und wobei die zweiten Hirnaktivitätssignale erfasst werden, während dieser einen Person die ein oder mehreren Fremdbilder (208-214) angezeigt werden;
   - Analyse (108) der erfassten ersten und zweiten Hirnaktivitätssignale durch die Authentifizierungssoftware (204), um die eine Person gegenüber der Authentifizierungssoftware zu authentifizieren, wobei die Authentifizierungssoftware die eine Person als erfolgreich authentifiziert behandelt, wenn:
      - die ersten Hirnaktivitätssignale sich signifikant unterscheiden von den zweiten Hirnaktivitätssignalen;
      - die zeitliche Reihenfolge der ersten und zweiten Hirnaktivitätssignale die in der Challenge codierte Reihenfolge von Selbstbildern und Fremdbildern wiedergibt;
      - alle ersten Hirnaktivitätssignale, die je während des Anzeigens eines der Selbstbilder erfasst werden, identisch oder hinreichend ähnlich zueinander sind; und
      - alle zweiten Hirnaktivitätssignale, die je während des Anzeigens eines der Fremdbilder erfasst werden, identisch oder hinreichend ähnlich zueinander sind.
2. Verfahren nach Klausel 1, wobei die Anzeige (216) aus einem Element besteht oder ein Element umfasst, wobei das Element ausgewählt ist aus einer Gruppe umfassend:
   - eine Vorrichtung (402) mit einem oder mehreren Spiegeln, welche dazu ausgebildet ist, ein Spiegelbild des Gesichts der einen Person oder ein Spiegelbild eines auf einem ID-Dokument der einen Person abgebildeten Gesichtsbildes optisch so zu erfassen und abzulenken, dass dieses Spiegelbild dieser Person als eines der ein oder mehreren Selbstbilder angezeigt wird;
   - eine digitale Anzeige, welche dazu ausgebildet ist, die mehreren Gesichtsbilder als digitale Bilder anzuzeigen.
3. Verfahren nach einer der vorigen Klauseln, ferner umfassend eine Bereitstellung der ein oder mehreren Selbstbilder für die Anzeige, wobei die Bereitstellung umfasst:
   - Erfassung und Ablenkung eines Spiegelbilds (404) des Gesichts der einen Person durch eine Vorrichtung mit einem oder mehreren Spiegeln, sodass dieses Spiegelbild dieser Person als eines der ein oder mehreren Selbstbilder angezeigt wird; oder
   - Erfassung und Ablenkung eines Spiegelbilds eines auf einem ID-Dokument der einen Person abgebildeten Gesichtsbildes durch eine Vorrichtung mit einem oder mehreren Spiegeln, sodass dieses Spiegelbild dieser Person als eines der ein oder mehreren Selbstbilder angezeigt wird; oder
   - Erfassung des Gesichts der einen Person (224) oder eines auf einem **ID-**Dokument (222, 300) der einen Person abgebildeten Gesichtsbildes (302) durch eine Kamera (502) in Form eines digitalen Gesichtsbildes (504) der einen Person.
4. Verfahren nach einer der vorigen Klauseln,
   - wobei die mehreren Gesichtsbilder mindestens zwei Selbstbilder (304, 404, 504) umfassen, wobei die mindestens zwei Selbstbildern auf die gleiche oder auf unterschiedliche Weise erfasst werden, wobei die Erfassungsweisen insbesondere ausgewählt sind aus den in Klausel 3 spezifizierten Erfassungsweisen.
5. Verfahren nach einer der vorigen Klauseln, wobei der KLHA-Sensor ein elektrischer Sensor ist.
6. Verfahren nach Klausel 5,
   - wobei der elektrische Sensor dazu ausgebildet ist, die ersten und/oder zweiten Hirnaktivitätssignale durch Messung von Ladungsänderungen und/oder von Ladungsumverteilungen zu erfassen; und/oder
   - wobei der elektrische Sensor dazu ausgebildet ist, die Amplitude der ersten und/oder zweiten Hirnaktivitätssignale und/oder die Änderung der Amplitude während eines Zeitintervalls zu erfassen.
7. Verfahren nach einer der vorigen Klauseln 5-6,
   - wobei die ersten Hirnaktivitätssignale identisch oder ähnlich sind zu den Hirnaktivitätssignalen gemäß Kurve 902 in Figur 9; und/oder
   - wobei die zweiten Hirnaktivitätssignale identisch oder ähnlich sind zu den Hirnaktivitätssignalen gemäß Kurve 904 in Figur 9.
8. Verfahren nach einer der vorigen Klauseln 5-7, wobei die Analyse (108) der erfassten Hirnaktivitätssignale umfasst:
   - Vergleich von Frequenz und Amplitudenverlauf der ersten (708, 712) Hirnaktivitätssignale und zweiten (704, 706, 710) Hirnaktivitätssignale untereinander, um festzustellen, ob sich die ersten Hirnaktivitätssignale signifikant von den zweiten Hirnaktivitätssignalen unterscheiden.
9. Verfahren nach einer der vorigen Klauseln 1-4, wobei der KLHA-Sensor ein optischer Sensor, insbesondere eine IR-Kamera oder IR-Videokamera oder eine Kamera oder Videokamera für Licht im sichtbaren Wellenlängenbereich ist.
10. Verfahren nach Klausel 9, wobei der KLHA-Sensor dazu ausgebildet ist, Bilder oder Bildsequenzen des Gesichts oder von Teilen des Gesichts der einen Person zu erfassen, die die ersten und/oder zweiten Hirnaktivitätssignale enthalten, wobei die Analyse der ersten und zweiten Hirnaktivitätssignale durch die Authentifizierungssoftware eine automatische Bildanalyse der Bilder oder Bildsequenzen umfasst, wobei die Bildanalyse umfasst:
   - Messung eines Pupillendurchmessers der einen Person; und/oder
   - Messung eines Pupillendurchmesseränderungsprofils der einen Person.
11. Verfahren nach einer der vorigen Klauseln 9-10,
   - wobei die ersten Hirnaktivitätssignale identisch oder ähnlich sind zu den Hirnaktivitätssignalen gemäß Kurve 952 in Figur 11; und/oder
   - wobei die zweiten Hirnaktivitätssignale identisch oder ähnlich sind zu den Hirnaktivitätssignalen gemäß Kurve 954 in Figur 11.
12. Verfahren nach einer der vorigen Klauseln 9-11, wobei die Analyse (108) der erfassten Hirnaktivitätssignale umfasst:
   - Bildverarbeitung zur Erkennung eines Pupillendurchmessers; und/oder
   - Machine-Learning zum Ermitteln von Referenz-Hirnaktivitätssignalen für die ersten und/oder zweiten Hirnaktivitätssignale; und/oder
   - Korrelation von Eigenschaften der ersten und zweiten Hirnaktivitätssignale mit den Zeiten, an welchen Selbstbilder und Fremdbilder angezeigt wurden.
13. Verfahren nach einem der vorigen Klauseln, wobei die Analyse (108) ausschließlich auf Basis der ersten und zweiten Hirnaktivitätssignalen und auf Basis der Sequenz der angezeigten Selbstbilder und Fremdbilder und optional zusätzlich auf Basis typisierter (nicht personen-individuell erhobener) Referenzdaten durchgeführt wird.
14. Verfahren nach einem der vorigen Klauseln, wobei das Verfahren außerdem die Löschung der erfassten Hirnaktivitätssignalen nach dem Authentifizierungsvorgang umfasst.
15. Authentifizierungssystem (200, 930) zur Authentifizierung einer Person (224), umfassend:
   - eine Anzeigevorrichtung (216);
   - eine Authentifizierungssoftware (204, 936), die konfiguriert ist zur:
      - Anzeige (104) mehrerer Gesichtsbilder (208-214, 304, 404, 504), die Gesichter mehrerer unterschiedlicher Personen abbilden, auf der Anzeigevorrichtung (216) für die eine Person (224), wobei die mehreren Gesichtsbilder ein oder mehrere Selbstbilder (304, 404, 504) und ein oder mehrere Fremdbilder umfassen, wobei ein Selbstbild ein Gesichtsbild dieser einen Person ist, wobei ein Fremdbild ein Gesichtsbild einer anderen der mehreren unterschiedlichen Personen ist, wobei das Anzeigen der mehreren Gesichtsbilder umfasst:
         ▪ für jeden Authentifizierungsvorgang, Erzeugung einer zufälligen Reihung (602) der mehreren Gesichtsbilder durch die Authentifizierungssoftware und Durchführen des Anzeigens der mehreren Gesichtsbilder in einer chronologischen Sequenz entsprechend der zu-fälligen Reihung; und/oder
         ▪ für jeden Authentifizierungsvorgang, zufällige Auswahl zumindest der ein oder mehreren Fremdbilder aus einer Gesichtsbilddatenbank (206) durch die Authentifizierungssoftware und Durchführen des Anzeigens der mehreren Gesichtsbilder einschließlich der ausgewählten Fremdbilder;
         wobei die für jeden Authentifizierungsvorgang erzeugte zufällige Reihung als Challenge dient;
   - einen Sensor (218) zur Sensierung von Hirnaktivitäten, wobei der Sensor (218) ein kontaktloser Sensor (218) ist, wobei der Sensor (218) nachfolgend als KLHA-Sensor (218) bezeichnet wird, wobei der Sensor konfiguriert ist zum:
      - während die mehreren Gesichtsbilder der einen Person angezeigt werden, Erfassung (106) von ersten (708, 712) und zweiten (704, 706, 710) Hirnaktivitätssignalen, die in der einen Person durch die Betrachtung der Gesichtsbilder jeweils ausgelöst werden, wobei die ersten Hirnaktivitätssignale erfasst werden, während dieser einen Person die ein oder mehreren Selbstbilder angezeigt werden, und wobei die zweiten Hirnaktivitätssignale erfasst werden, während dieser einen Person die ein oder mehreren Fremdbilder angezeigt werden;
   - wobei die Authentifizierungssoftware ferner konfiguriert ist zur:
      - Analyse (108) der erfassten ersten und zweiten Hirnaktivitätssignale, um die eine Person gegenüber der Authentifizierungssoftware zu authentifizieren, wobei die Authentifizierungssoftware die eine Person als erfolgreich authentifiziert behandelt, wenn:
         ▪ die ersten Hirnaktivitätssignale sich signifikant unterscheiden von den zweiten Hirnaktivitätssignalen;
         ▪ die zeitliche Reihenfolge der ersten und zweiten Hirnaktivitätssignale die in der Challenge codierte Reihenfolge von Selbstbildern und Fremdbildern wiedergibt;
         ▪ alle ersten Hirnaktivitätssignale, die je während des Anzeigens eines der Selbstbilder erfasst werden, identisch oder hinreichend ähnlich zueinander sind; und
         ▪ alle zweiten Hirnaktivitätssignale, die je während des Anzeigens eines der Fremdbilder erfasst werden, identisch oder hinreichend ähnlich zueinander sind.
16. Authentifizierungssystem nach Klausel 15, ferner umfassend ein Terminal, wobei die Anzeigevorrichtung Bestandteil des Terminals ist und wobei das Terminal insbesondere ein Flughafenterminal, ein Grenzkontrollterminal, ein Terminal zur Kontrolle des Zutritts zu einem geschützten geographischen Bereich ist.

### Bezugszeichenliste

- 102-108: Schritte
- 200: Authentifizierungssystem
- 201: Terminal
- 202: Datenspeicher
- 204: Authentifizierungssoftware
- 206: Bilddatenbank
- 208: Gesichtsbild (Fremdbild)
- 210: Gesichtsbild (Fremdbild)
- 212: Gesichtsbild (Fremdbild)
- 214: Gesichtsbild (Fremdbild)
- 216: Anzeige
- 218: KLHA-Sensor(en)
- 220: Dokumenteneinzug
- 222: Dokument
- 224: Person
- 226: Prozessor(en)
- 300: Authentifizierungssystem
- 302: Portraitbild auf Dokument
- 304: Gesichtsbild (Selbstbild)
- 400: Authentifizierungssystem
- 402: Spiegelvorrichtung
- 404: Gesichtsbild (Selbstbild)
- 500: Authentifizierungssystem
- 502: Bilderfassungseinheit (Kamera)
- 504: Gesichtsbild (Selbstbild)
- 602: Sequenz Gesichtsbilder
- 702: Sequenz Hirnaktivitätssignale
- 704: zweite Hirnaktivitätssignale
- 706: zweite Hirnaktivitätssignale
- 708: erste Hirnaktivitätssignale
- 710: zweite Hirnaktivitätssignale
- 712: erste Hirnaktivitätssignale
- 800: Authentifizierungssoftware auf Basis eines neuronalen Netzes
- 802: Vorverarbeitung Modul
- 804: Rekurrentes neuronales Netzwerk
- 806: Auswertungsmodul
- 900: Signalplot erfasst von elektrischem Sensor
- 902: erstes Hirnaktivitätssignal (bei Selbstbildanzeige)
- 904: zweites Hirnaktivitätssignal (bei Fremdbildanzeige)
- 930: Authentifizierungssystem
- 934: Anzeigemodul
- 936: Analysemodul
- 938: Drittsystem
- 950: Signalplot erfasst von optischem Sensor
- 952: erstes Hirnaktivitätssignal (bei Selbstbildanzeige)
- 954: zweites Hirnaktivitätssignal (bei Fremdbildanzeige)

## Patentansprüche

1. Verfahren zur Authentifizierung einer Person (224), umfassend:
- Bereitstellung (102) einer Anzeigevorrichtung (216);
- Bereitstellung (103) eines Sensors (218) zur Sensierung von Hirnaktivitäten, wobei der Sensor (218) ein kontaktloser Sensor (218) ist, wobei der Sensor (218) nachfolgend als KLHA-Sensor (218) bezeichnet wird;
- Anzeige (104) mehrerer Gesichtsbilder (208-214,304, 404, 504), die Gesichter mehrerer unterschiedlicher Personen abbilden, auf der Anzeigevorrichtung für die eine Person (224), wobei die mehreren Gesichtsbilder ein oder mehrere Selbstbilder (304, 404, 504) und ein oder mehrere Fremdbilder umfassen, wobei ein Selbstbild ein Gesichtsbild dieser einen Person ist, wobei ein Fremdbild ein Gesichtsbild einer anderen der mehreren unterschiedlichen Personen ist, wobei das Anzeigen der mehreren Gesichtsbilder umfasst:
• für jeden Authentifizierungsvorgang, Erzeugung einer zufälligen Reihung (602) der mehreren Gesichtsbilder durch die Authentifizierungssoftware und Durchführen des Anzeigens der mehreren Gesichtsbilder in einer chronologischen Sequenz entsprechend der zufälligen Reihung; und/oder
• für jeden Authentifizierungsvorgang, zufällige Auswahl zumindest der ein oder mehreren Fremdbilder aus einer Gesichtsbilddatenbank (206) durch die Authentifizierungssoftware und Durchführen des Anzeigens der mehreren Gesichtsbilder einschließlich der ausgewählten Fremdbilder;
wobei die für jeden Authentifizierungsvorgang erzeugte zufällige Reihung als Challenge dient;
- während die mehreren Gesichtsbilder der einen Person angezeigt werden, Erfassung (106) von ersten (708, 712, 902) und zweiten (704, 706, 710, 904) Hirnaktivitätssignalen, die in der einen Person durch die Betrachtung der Gesichtsbilder jeweils ausgelöst werden, durch den Sensor, wobei die ersten Hirnaktivitätssignale erfasst werden, während dieser einen Person die ein oder mehreren Selbstbilder (304, 404, 504) angezeigt werden, und wobei die zweiten Hirnaktivitätssignale erfasst werden, während dieser einen Person die ein oder mehreren Fremdbilder (208-214) angezeigt werden;
- Analyse (108) der erfassten ersten und zweiten Hirnaktivitätssignale durch die Authentifizierungssoftware (204), um die eine Person gegenüber der Authentifizierungssoftware zu authentifizieren, wobei die Authentifizierungssoftware die eine Person als erfolgreich authentifiziert behandelt, wenn:
• die ersten Hirnaktivitätssignale sich signifikant unterscheiden von den zweiten Hirnaktivitätssignalen;
• die zeitliche Reihenfolge der ersten und zweiten Hirnaktivitätssignale die in der Challenge codierte Reihenfolge von Selbstbildern und Fremdbildern wiedergibt;
• alle ersten Hirnaktivitätssignale, die je während des Anzeigens eines der Selbstbilder erfasst werden, identisch oder hinreichend ähnlich zueinander sind; und
• alle zweiten Hirnaktivitätssignale, die je während des Anzeigens eines der Fremdbilder erfasst werden, identisch oder hinreichend ähnlich zueinander sind.

2. Verfahren nach Anspruch 1, wobei die Anzeigevorrichtung (216) aus einem Element besteht oder ein Element umfasst, wobei das Element ausgewählt ist aus einer Gruppe umfassend:
- eine Vorrichtung (402) mit einem oder mehreren Spiegeln, welche dazu ausgebildet ist, ein Spiegelbild des Gesichts der einen Person oder ein Spiegelbild eines auf einem ID-Dokument der einen Person abgebildeten Gesichtsbildes optisch so zu erfassen und abzulenken, dass dieses Spiegelbild dieser Person als eines der ein oder mehreren Selbstbilder angezeigt wird;
- eine digitale Anzeige, welche dazu ausgebildet ist, die mehreren Gesichtsbilder als digitale Bilder anzuzeigen.

3. Verfahren nach einem der vorigen Ansprüche, ferner umfassend eine Bereitstellung der ein oder mehreren Selbstbilder für die Anzeige, wobei die Bereitstellung umfasst:
- Erfassung und Ablenkung eines Spiegelbilds (404) des Gesichts der einen Person durch eine Vorrichtung mit einem oder mehreren Spiegeln, sodass dieses Spiegelbild dieser Person als eines der ein oder mehreren Selbstbilder angezeigt wird; oder
- Erfassung und Ablenkung eines Spiegelbilds eines auf einem ID-Dokument der einen Person abgebildeten Gesichtsbildes durch eine Vorrichtung mit einem oder mehreren Spiegeln, sodass dieses Spiegelbild dieser Person als eines der ein oder mehreren Selbstbilder angezeigt wird; oder
- Erfassung des Gesichts der einen Person (224) oder eines auf einem ID-Dokument (222, 300) der einen Person abgebildeten Gesichtsbildes (302) durch eine Kamera (502) in Form eines digitalen Gesichtsbildes (504) der einen Person.

4. Verfahren nach einem der vorigen Ansprüche,
- wobei die mehreren Gesichtsbilder mindestens zwei Selbstbilder (304, 404, 504) umfassen, wobei die mindestens zwei Selbstbildern auf die gleiche oder auf unterschiedliche Weise erfasst werden, wobei die Erfassungsweisen insbesondere ausgewählt sind aus den in Anspruch spezifizierten Erfassungsweisen.

5. Verfahren nach einem der vorigen Ansprüche, wobei der KLHA-Sensor ein elektrischer Sensor ist.

6. Verfahren nach Anspruch 5, wobei die Analyse (108) der erfassten Hirnaktivitätssignale umfasst:
- Vergleich von Frequenz und Amplitudenverlauf der ersten (708, 712) Hirnaktivitätssignale und zweiten (704, 706, 710) Hirnaktivitätssignale untereinander, um festzustellen, ob sich die ersten Hirnaktivitätssignale signifikant von den zweiten Hirnaktivitätssignalen unterscheiden.

7. Verfahren nach einem der vorigen Ansprüche 1-4, wobei der KLHA-Sensor ein Sensor (218) ist, welcher ein oder mehrere Signale empfangen kann, die von einem Menschen ausgesendet werden, ohne dass während des Empfangs der Signale ein Kontakt des Sensors (218) zu dem Menschen besteht, wobei die empfangenen Signale direkt oder indirekt Aufschluss über Hirnaktivitäten des Menschen geben, wobei der KLHA-Sensor ein optischer Sensor, insbesondere eine IR-Kamera oder IR-Videokamera oder eine Kamera oder Videokamera für Licht im sichtbaren Wellenlängenbereich ist, und wobei die ersten und zweiten Hirnaktivitätssignale von der einen Person ausgestrahlte Signale sind, welche durch eine Hirnaktivität direkt oder indirekt bewirkt werden.

8. Verfahren nach Anspruch 7, wobei der KLHA-Sensor dazu ausgebildet ist, Bilder oder Bildsequenzen des Gesichts oder von Teilen des Gesichts der einen Person zu erfassen, die die ersten und/oder zweiten Hirnaktivitätssignale enthalten, wobei die Analyse der ersten und zweiten Hirnaktivitätssignale durch die Authentifizierungssoftware eine automatische Bildanalyse der Bilder oder Bildsequenzen umfasst, wobei die Bildanalyse umfasst:
- Messung eines Pupillendurchmessers der einen Person; und/oder
- Messung eines Pupillendurchmesseränderungsprofils der einen Person.

9. Verfahren nach einem der vorigen Ansprüche, wobei die Analyse (108) ausschließlich auf Basis der ersten und zweiten Hirnaktivitätssignalen und auf Basis der Sequenz der angezeigten Selbstbilder und Fremdbilder durchgeführt wird.

10. Verfahren nach einem der vorigen Ansprüche, wobei das Verfahren außerdem die Löschung der erfassten Hirnaktivitätssignalen nach dem Authentifizierungsvorgang umfasst.

11. Authentifizierungssystem (200, 930) zur Authentifizierung einer Person (224), umfassend:
- eine Anzeigevorrichtung (216);
- eine Authentifizierungssoftware (204, 936), die konfiguriert ist zur:
• Anzeige (104) mehrerer Gesichtsbilder (208-214, 304, 404, 504), die Gesichter mehrerer unterschiedlicher Personen abbilden, auf der Anzeigevorrichtung (216) für die eine Person (224), wobei die mehreren Gesichtsbilder ein oder mehrere Selbstbilder (304, 404, 504) und ein oder mehrere Fremdbilder umfassen, wobei ein Selbstbild ein Gesichtsbild dieser einen Person ist, wobei ein Fremdbild ein Gesichtsbild einer anderen der mehreren unterschiedlichen Personen ist, wobei das Anzeigen der mehreren Gesichtsbilder umfasst:
▪ für jeden Authentifizierungsvorgang, Erzeugung einer zufälligen Reihung (602) der mehreren Gesichtsbilder durch die Authentifizierungssoftware und Durchführen des Anzeigens der mehreren Gesichtsbilder in einer chronologischen Sequenz entsprechend der zu-fälligen Reihung; und/oder
▪ für jeden Authentifizierungsvorgang, zufällige Auswahl zumindest der ein oder mehreren Fremdbilder aus einer Gesichtsbilddatenbank (206) durch die Authentifizierungssoftware und Durchführen des Anzeigens der mehreren Gesichtsbilder einschließlich der ausgewählten Fremdbilder;
wobei die für jeden Authentifizierungsvorgang erzeugte zufällige Reihung als Challenge dient;
- einen Sensor (218) zur Sensierung von Hirnaktivitäten, wobei der Sensor (218) ein kontaktloser Sensor (218) ist, wobei der Sensor (218) nachfolgend als KLHA-Sensor (218) bezeichnet wird, wobei der Sensor konfiguriert ist zum:
• während die mehreren Gesichtsbilder der einen Person angezeigt werden, Erfassung (106) von ersten (708, 712) und zweiten (704, 706, 710) Hirnaktivitätssignalen, die in der einen Person durch die Betrachtung der Gesichtsbilder jeweils ausgelöst werden, wobei die ersten Hirnaktivitätssignale erfasst werden, während dieser einen Person die ein oder mehreren Selbstbilder angezeigt werden, und wobei die zweiten Hirnaktivitätssignale erfasst werden, während dieser einen Person die ein oder mehreren Fremdbilder angezeigt werden;
- wobei die Authentifizierungssoftware ferner konfiguriert ist zur:
• Analyse (108) der erfassten ersten und zweiten Hirnaktivitätssignale, um die eine Person gegenüber der Authentifizierungssoftware zu authentifizieren, wobei die Authentifizierungssoftware die eine Person als erfolgreich authentifiziert behandelt, wenn:
▪ die ersten Hirnaktivitätssignale sich signifikant unterscheiden von den zweiten Hirnaktivitätssignalen;
▪ die zeitliche Reihenfolge der ersten und zweiten Hirnaktivitätssignale die in der Challenge codierte Reihenfolge von Selbstbildern und Fremdbildern wiedergibt;
▪ alle ersten Hirnaktivitätssignale, die je während des Anzeigens eines der Selbstbilder erfasst werden, identisch oder hinreichend ähnlich zueinander sind; und
▪ alle zweiten Hirnaktivitätssignale, die je während des Anzeigens eines der Fremdbilder erfasst werden, identisch oder hinreichend ähnlich zueinander sind.

12. Authentifizierungssystem nach Anspruch 11, ferner umfassend ein Terminal, wobei die Anzeigevorrichtung Bestandteil des Terminals ist und wobei das Terminal insbesondere ein Flughafenterminal, ein Grenzkontrollterminal, ein Terminal zur Kontrolle des Zutritts zu einem geschützten geographischen Bereich ist.

## Claims

1. A method for authenticating a person (224), comprising:
- providing (102) a display device (216);
- providing (103) a sensor (218) for sensing brain activity, wherein the sensor (218) is a contactless sensor (218), wherein the sensor (218) is hereinafter referred to as a **KLHA** sensor (218);
- displaying (104) a plurality of facial images (208-214, 304, 404, 504) depicting the faces of a plurality of different persons on the display device for said one person (224), wherein the plurality of facial images comprises one or more self-images (304, 404, 504) and one or more third-party images, wherein a self-image is a facial image of said one person, wherein a third-party image is a facial image of another of the plurality of different persons, wherein the displaying of the plurality of facial images comprises:
• for each authentication process, generating a random order (602) of the plurality of facial images by the authentication software and displaying the plurality of facial images in a chronological sequence corresponding to the random order; and/or
• for each authentication process, randomly selecting at least the one or more other-person images from a facial image database (206) by the authentication software and displaying the plurality of facial images including the selected other-person images;
wherein the random order generated for each authentication process serves as a challenge;
- whilst the multiple facial images of the one person are being displayed, capturing (106) of first (708, 712, 902) and second (704, 706, 710, 904) brain activity signals, each triggered in said one person by viewing the facial images, by the sensor, wherein the first brain activity signals are captured whilst the one or more self-images (304, 404, 504) are displayed to said one person, and wherein the second brain activity signals are captured whilst the one person is shown the one or more third-party images (208-214);
- analyzing (108) of the captured first and second brain activity signals by the authentication software (204) to authenticate the one person to the authentication software, wherein the authentication software treats the one person as successfully authenticated if:
• the first brain activity signals differ significantly from the second brain activity signals;
• the temporal sequence of the first and second brain activity signals reflects the sequence of self-images and other-person images encoded in the challenge;
• all first brain activity signals ever captured during the display of any of the self-images are identical or sufficiently similar to one another; and
• all second brain activity signals ever captured during the display of any of the other-person images are identical or sufficiently similar to one another.

2. A method according to claim 1, wherein the display device (216) consists of or comprises an element, wherein the element is selected from a group comprising:
- a device (402) having one or more mirrors, which is configured to optically capture and deflect a mirror image of the face of the one person or a mirror image of a facial image depicted on an ID document of the one person in such a way that this mirror image of this person is displayed as one of the one or more self-images;
- a digital display configured to display the plurality of facial images as digital images.

3. A method according to one of the preceding claims, further comprising providing the one or more self-images for display, wherein the providing comprises:
- capturing and deflecting a mirror image (404) of the face of the one person by a device comprising one or more mirrors, such that this mirror image of this person is displayed as one of the one or more self-images; or
- capturing and redirecting a mirror image of a facial image depicted on an ID document of said one person by means of a device comprising one or more mirrors, such that said mirror image of said person is displayed as one of the one or more self-images; or
- capturing the face of the person (224) or a facial image (302) depicted on an ID document (222, 300) of the person by a camera (502) in the form of a digital facial image (504) of the person.

4. A method according to any of the preceding claims,
- wherein the plurality of facial images comprises at least two self-images (304, 404, 504), wherein the at least two self-images are captured in the same or in different ways, wherein the capturing methods are, in particular, selected from the capturing methods specified in claim 3.

5. A method according to one of the preceding claims, wherein the KLHA sensor is an electrical sensor.

6. A method according to claim 5, wherein the analyzing (108) of the captured brain activity signals comprises:
- comparing the frequency and amplitude profiles of the first (708, 712) brain activity signals and the second (704, 706, 710) brain activity signals with one another to determine whether the first brain activity signals differ significantly from the second brain activity signals.

7. A method according to any one of the preceding claims 1-4, wherein the KLHA sensor is a sensor (218) capable of receiving one or more signals emitted by a human being without the sensor (218) being in contact with the human being during the reception of the signals, wherein the received signals provide direct or indirect information about the human being's brain activity, wherein the KLHA sensor is an optical sensor, in particular an IR camera or IR video camera or a camera or video camera for light in the visible wavelength range, and wherein the first and second brain activity signals are signals emitted by the person, which are directly or indirectly caused by brain activity.

8. A method according to claim 7, wherein the **KLHA** sensor is configured to capture images or image sequences of the face or parts of the face of the one person that contain the first and/or second brain activity signals, wherein the analyzing of the first and second brain activity signals by the authentication software comprises an automatic image analysis of the images or image sequences, wherein the image analysis comprises:
- measuring a pupil diameter of the person; and/or
- measuring a pupil diameter change profile of the person.

9. A method according to one of the preceding claims, wherein the analyzing (108) is performed exclusively on the basis of the first and second brain activity signals and on the basis of the sequence of the displayed self-images and third-party images.

10. A method according to one of the preceding claims, wherein the method further comprises deleting the captured brain activity signals after the authentication process.

11. An authentication system (200, 930) for authenticating a person (224), comprising:
- a display device (216);
- authentication software (204, 936) configured to:
• displaying (104) a plurality of facial images (208-214, 304, 404, 504) depicting the faces of a plurality of different persons on the display device (216) to the one person (224), wherein the plurality of facial images comprises one or more self-images (304, 404, 504) and one or more third-party images, wherein a self-image is a facial image of said one person, wherein a third-party image is a facial image of another of the plurality of different persons, wherein the displaying of the plurality of facial images comprises:
▪ for each authentication process, generating a random order (602) of the plurality of facial images by the authentication software and displaying the plurality of facial images in a chronological sequence corresponding to the random order; and/or
▪ for each authentication process, randomly selecting at least one or more of the other-person images from a facial image database (206) by the authentication software and displaying the plurality of facial images including the selected other-person images;
wherein the random order generated for each authentication process serves as a challenge;
- a sensor (218) for sensing brain activity, wherein the sensor (218) is a contactless sensor (218), wherein the sensor (218) is hereinafter referred to as a **KLHA** sensor (218), wherein the sensor is configured to:
• whilst the multiple facial images of the one person are being displayed, capturing (106) of first (708, 712) and second (704, 706, 710) brain activity signals triggered in said one person by viewing the facial images, respectively, wherein the first brain activity signals are captured whilst the one or more self-images are displayed to said one person, and wherein the second brain activity signals are captured whilst the one or more other-person images are displayed to said one person;
- wherein the authentication software is further configured to:
• analyzing (108) the captured first and second brain activity signals to authenticate the one person to the authentication software, wherein the authentication software treats the one person as successfully authenticated if:
▪ the first brain activity signals differ significantly from the second brain activity signals;
▪ the temporal sequence of the first and second brain activity signals reflects the sequence of self-images and other-person images encoded in the challenge;
▪ all first brain activity signals ever captured whilst displaying one of the self-images are identical or sufficiently similar to one another; and
▪ all second brain activity signals ever captured during the display of any of the third-party images are identical or sufficiently similar to one another.

12. An authentication system according to claim 11, further comprising a terminal, wherein the display device forms part of the terminal and wherein the terminal is, in particular, an airport terminal, a border control terminal, or a terminal for controlling access to a protected geographical area.

## Revendications

1. Procédé d'authentification d'une personne (224), comprenant :
- la fourniture (102) d'un dispositif d'affichage (216) ;
- la fourniture (103) d'un capteur (218) pour détecter une activité cérébrale, dans lequel le capteur (218) est un capteur sans contact (218), dans lequel le capteur (218) est appelé ci-après capteur KLHA (218) ;
- l'affichage (104) d'une pluralité d'images faciales (208-214, 304, 404, 504) représentant des visages d'une pluralité de personnes différentes sur le dispositif d'affichage pour la personne (224), dans lequel la pluralité d'images faciales comprend une ou plusieurs images personnelles (304, 404, 504) et une ou plusieurs images étrangères, dans lequel une image personnelle est une image faciale de cette personne, dans lequel une image étrangère est une image faciale d'une autre de la pluralité de personnes différentes, dans lequel l'affichage de la pluralité d'images faciales comprend :
• pour chaque opération d'authentification, la génération d'un classement aléatoire (602) de la pluralité d'images faciales par le logiciel d'authentification et la réalisation de l'affichage de la pluralité d'images faciales dans une séquence chronologique correspondant au classement aléatoire ; et/ou
• pour chaque opération d'authentification, la sélection aléatoire d'au moins la ou les images étrangères à partir d'une base de données d'images faciales (206) par le logiciel d'authentification et la réalisation de l'affichage de la pluralité d'images faciales comprenant les images étrangères sélectionnées ;
dans lequel le classement aléatoire généré pour chaque opération d'authentification sert de défi ;
- tandis que la pluralité d'images faciales de la personne sont affichées, l'acquisition (106), par le capteur, de premiers (708, 712, 902) et seconds (704, 706, 710, 904) signaux d'activité cérébrale déclenchés dans la personne par la visualisation des images faciales, respectivement, dans lequel les premiers signaux d'activité cérébrale sont acquis tandis que la ou les images personnelles (304, 404, 504) sont affichées à cette personne et dans lequel les seconds signaux d'activité cérébrale sont acquis tandis que cette personne se voit afficher la ou les images étrangères (208-214) ;
- l'analyse (108), par le logiciel d'authentification (204), des premiers et seconds signaux d'activité cérébrale acquis pour authentifier la personne auprès du logiciel d'authentification, dans lequel le logiciel d'authentification traite la personne comme authentifiée avec succès si :
• les premiers signaux d'activité cérébrale sont significativement différents des seconds signaux d'activité cérébrale ;
• l'ordre chronologique des premiers et seconds signaux d'activité cérébrale reflète la séquence d'images personnelles et d'images étrangères codée dans le défi;
• tous les premiers signaux d'activité cérébrale acquis chacun pendant l'affichage de l'une des images personnelles sont identiques ou suffisamment similaires les uns aux autres ; et
• tous les seconds signaux d'activité cérébrale acquis chacun pendant l'affichage de l'une des images étrangères sont identiques ou suffisamment similaires les uns aux autres.

2. Procédé selon la revendication 1, dans lequel le dispositif d'affichage (216) est constitué d'un élément ou comprend un élément, dans lequel l'élément est choisi dans un groupe comprenant :
- un dispositif (402) avec un ou plusieurs miroirs configuré pour acquérir et défléchir optiquement une image miroir du visage de la personne ou une image miroir d'une image faciale représentée sur un document d'identification de la personne de sorte que cette image miroir de cette personne soit affichée comme l'une de la ou des images personnelles ;
- un affichage numérique configuré pour afficher la pluralité d'images faciales comme des images numériques.

3. Procédé selon l'une quelconque des revendications précédentes, comprenant en outre la fourniture de la ou des images personnelles pour l'affichage, dans lequel la fourniture comprend :
- l'acquisition et la déflexion d'une image miroir (404) du visage de la personne par un dispositif avec un ou plusieurs miroirs de sorte que cette image miroir de cette personne soit affichée comme l'une de la ou des images personnelles ; ou
- l'acquisition et la déflexion d'une image miroir d'une image faciale représentée sur un document d'identification de la personne par un dispositif avec un ou plusieurs miroirs de sorte que cette image miroir de cette personne soit affichée comme l'une de la ou des images personnelles ; ou
- l'acquisition du visage de la personne (224) ou d'une image faciale (302) représentée sur un document d'identification (222, 300) de la personne par une caméra (502) sous la forme d'une image faciale numérique (504) de la personne.

4. Procédé selon l'une quelconque des revendications précédentes,
- dans lequel la pluralité d'images faciales comprend au moins deux images personnelles (304, 404, 504), dans lequel les au moins deux images personnelles sont acquises de la même manière ou de manière différente, dans lequel les manières d'acquisition sont en particulier choisies parmi les manières d'acquisition spécifiées dans la revendication 3.

5. Procédé selon l'une quelconque des revendications précédentes, dans lequel le capteur KLHA est un capteur électrique.

6. Procédé selon la revendication 5, dans lequel l'analyse (108) des signaux d'activité cérébrale acquis comprend :
- la comparaison des profils de fréquence et d'amplitude des premiers (708, 712) signaux d'activité cérébrale et des seconds (704, 706, 710) signaux d'activité cérébrale entre eux pour déterminer si les premiers signaux d'activité cérébrale sont significativement différents des seconds signaux d'activité cérébrale.

7. Procédé selon l'une quelconque des revendications précédentes 1 à 4, dans lequel le capteur KLHA est un capteur (218) qui peut recevoir un ou plusieurs signaux émis par un être humain sans qu'il n'y ait de contact du capteur (218) avec l'être humain pendant la réception des signaux, dans lequel les signaux reçus fournissent directement ou indirectement des informations sur l'activité cérébrale de l'être humain, dans lequel le capteur KLHA est un capteur optique, en particulier une caméra IR ou une caméra vidéo IR ou une caméra ou une caméra vidéo pour une lumière dans la plage de longueurs d'onde visible, et dans lequel les premiers et seconds signaux d'activité cérébrale sont des signaux émis par la personne qui sont directement ou indirectement provoqués par une activité cérébrale.

8. Procédé selon la revendication 7, dans lequel le capteur KLHA est configuré pour acquérir des images ou des séquences d'images du visage ou de parties du visage de la personne contenant les premiers et/ou seconds signaux d'activité cérébrale, dans lequel l'analyse des premiers et seconds signaux d'activité cérébrale par le logiciel d'authentification comprend une analyse d'image automatique des images ou des séquences d'images, dans lequel l'analyse d'image comprend :
- la mesure d'un diamètre de pupille de la personne ; et/ou
- la mesure d'un profil de changement de diamètre de pupille de la personne.

9. Procédé selon l'une quelconque des revendications précédentes, dans lequel l'analyse (108) est réalisée uniquement sur la base des premiers et seconds signaux d'activité cérébrale et sur la base de la séquence d'images personnelles et d'images étrangères affichées.

10. Procédé selon l'une quelconque des revendications précédentes, dans lequel le procédé comprend en outre la suppression des signaux d'activité cérébrale acquis après l'opération d'authentification.

11. Système d'authentification (200, 930) pour authentifier une personne (224), comprenant :
- un dispositif d'affichage (216) ;
- un logiciel d'authentification (204, 936) configuré pour :
• afficher (104) une pluralité d'images faciales (208-214, 304, 404, 504) représentant des visages d'une pluralité de personnes différentes sur le dispositif d'affichage (216) pour la personne (224), dans lequel la pluralité d'images faciales comprend une ou plusieurs images personnelles (304, 404, 504) et une ou plusieurs images étrangères, dans lequel une image personnelle est une image faciale de cette personne, dans lequel une image étrangère est une image faciale d'une autre de la pluralité de personnes différentes, dans lequel l'affichage de la pluralité d'images faciales comprend :
▪ pour chaque opération d'authentification, la génération d'un classement aléatoire (602) de la pluralité d'images faciales par le logiciel d'authentification et la réalisation de l'affichage de la pluralité d'images faciales dans une séquence chronologique correspondant au classement aléatoire ; et/ou
▪ pour chaque opération d'authentification, la sélection aléatoire d'au moins la ou les images étrangères à partir d'une base de données d'images faciales (206) par le logiciel d'authentification et la réalisation de l'affichage de la pluralité d'images faciales comprenant les images étrangères sélectionnées ;
dans lequel le classement aléatoire généré pour chaque opération d'authentification sert de défi ;
- un capteur (218) pour détecter une activité cérébrale, dans lequel le capteur (218) est un capteur sans contact (218), dans lequel le capteur (218) est appelé ci-après capteur KLHA (218), dans lequel le capteur est configuré pour :
• tandis que la pluralité d'images faciales de la personne sont affichées, acquérir (106) des premiers (708, 712) et seconds (704, 706, 710) signaux d'activité cérébrale déclenchés dans la personne par la visualisation des images faciales, respectivement, dans lequel les premiers signaux d'activité cérébrale sont acquis tandis que la ou les images personnelles sont affichées à cette personne et dans lequel les seconds signaux d'activité cérébrale sont acquis tandis que cette personne se voit afficher la ou les images étrangères;
- dans lequel le logiciel d'authentification est en outre configuré pour :
• analyser (108) les premiers et seconds signaux d'activité cérébrale acquis pour authentifier la personne auprès du logiciel d'authentification, dans lequel le logiciel d'authentification traite la personne comme authentifiée avec succès si :
▪ les premiers signaux d'activité cérébrale sont significativement différents des seconds signaux d'activité cérébrale ;
▪ l'ordre chronologique des premiers et seconds signaux d'activité cérébrale reflète la séquence d'images personnelles et d'images étrangères codée dans le défi;
▪ tous les premiers signaux d'activité cérébrale acquis chacun pendant l'affichage de l'une des images personnelles sont identiques ou suffisamment similaires les uns aux autres ; et
▪ tous les seconds signaux d'activité cérébrale acquis chacun pendant l'affichage de l'une des images étrangères sont identiques ou suffisamment similaires les uns aux autres.

12. Système d'authentification selon la revendication 11, comprenant en outre un terminal, dans lequel le dispositif d'affichage fait partie du terminal et dans lequel le terminal est en particulier un terminal d'aéroport, un terminal de contrôle de frontière, un terminal de contrôle d'accès à une zone géographique protégée.
